# EUROPEAN PATENT APPLICATION

(11) **EP 1 701 165 A1**
(43) Date of publication of application: **13.09.2006**
(21) Application number: 05004930.3
(22) Date of filing: 07.03.2005
(51) Int. Cl.: G01N 33/574, G01N 33/68

(54) **Therapeutic and diagnostic uses of TKTL1 and inhibitors and activators thereof**

(71) Applicant: Coy, Johannes, Dr., 64853 Otzberg (DE)
(72) Inventor: Coy, Johannes, Dr., 64853 Otzberg (DE)
(74) Representative: Rudolph, Ulrike

(57) **Abstract**

The present invention relates to methods for treatment, detection and diagnosis of disorders associated with an altered transketolase (transketolase-like 1; TKTL1) with a modified substrate specificity and modified reaction modus. Different protein isoforms of the altered transketolase are present in enzymatically active or inactive forms. Dependent on the localization of the protein isoforms within the cell, the aggregation status, and the dimerization, the functions of the altered transketolase protein isoforms change. In particular, the present invention relates to methods for the detection and/or treatment of diseases associated with a reduced or enhanced enzymatic (trans-)ketolase activity, a different localization within the cell, a different aggregation status, or a different dimerization in biological samples.

## Description

The present invention relates to methods for treatment, detection and diagnosis of disorders associated with an altered transketolase (transketolase-like 1; TKTL1) with a modified substrate specificity and modified reaction modus. Different protein isoforms of the altered transketolase are present in enzymatically active or inactive forms. Dependent on the localization of the protein isoforms within the cell, the aggregation status, and the dimerization, the functions of the altered transketolase protein isoforms change. In particular, the present invention relates to methods for the detection and/or treatment of diseases associated with a reduced or enhanced enzymatic (trans-)ketolase activity, a different localization within the cell, a different aggregation status, or a different dimerization in biological samples.

The pentose phosphate pathway (PPP), also known as the phosphogluconate pathway or the hexose monophosphate shunt, represents a multifunctional pathway specialized to carry out three main activities, depending on the cell type and its metabolic state. The first function of the pentose pathway in mammalian cells is the generation of reducing power in the form of NADPH through a carbon flow from hexoses (mainly glucose) to pentoses which are recycled back into glycolysis through the non-oxidative pathway. The second function is the complete oxidative degradation of pentoses by converting them into hexoses, which can enter the glycolytic sequence. The third function of the PPP is to convert hexoses into pentoses, particularly D-ribose 5-phosphate, required in the synthesis of nucleic acids.

The non-oxidative pentose phosphate pathway is controlled by transketolase enzyme reactions. Up to now all biochemical or molecular results regarding transketolase proteins/enzymes have been interpreted as the result of the TKT gene although three transketolase(-like) genes (TKT, TKTL1 and TKTL2) are present in humans. The transketolase (TKT) gene was cloned by Abedina et al. (Biophys. Res. Commun. 183, 1159-1166, 1992) and McCool et al. (J. Biol. Chem. 268, 1397-1404, 1993) and is located at chromosomal band 3p21. The transketolase-like-1 gene (TKTL1) was identified in the chromosomal band Xq28 by Coy et al. (Genomics 32, 309-316, 1996). A cDNA sequence encoding a putative transketolase-like 2 (TKTL2) protein has been submitted to gene bank (acc. no. CR533560). TKTL2 gene sequences are present on human bac clone RP11-218F10 (Acc. No. AC108154) and the clone is mapped to chromosome 4 (4q32.2).

The TKTL1 gene was originally seen as a pseudogene since the third putative coding exon harbours a stop codon. In contrast to this the actual TKTL1 transcript missed the stop codon harboring exon. Since the deletion of the exon with the putative stop codon does not change the frame of the transcript, an open reading frame encoding a putative TKTL1 protein homologous to the TKT protein over the entire length has been detected indicating that the TKTL1 gene is still an active and coding gene. During the evolution of the vertebrate genome a mutation has occurred before the human and murine lineages diverged leading to a deleted TKTL1 protein. The mutation within the TKTL1 transketolase has removed amino acid residues determining substrate specifity and reaction modus.

Why is the transketolase enzyme reaction so important for a tumor cell? One reason is the generation of ribose for DNA and RNA synthesis. Another reason is, in combination with other enzyme reactions of the oxidative and non-oxidative PPP, the generation of NADPH as a reducing agent for synthesis. A further, although not well recognized, aspect of the transketolase enzyme reaction is that it links glucose degradation with lactate production.

Lee et al. (Am. J. Physiol. 274, 843-851, 1998) analysed the metabolism of [1,2-¹³C₂]glucose in HepG2 cells by glucose-6-phosphate dehydrogenase, transketolase and transaldolase enzyme reactions by determining pentose and lactate isotopomers with either one or two ¹³C substitutions. [1,2-¹³C₂]glucose is converted to singly labelled lactate [1-¹³C₂]lactate, [2-¹³C₂]lactate, [3-¹³C₂] lactate by the PPP whereas doubly labelled [2,3-¹³C₂]lactate is produced by the Embden Meyerhof pathway (glycolytic pathway). After 24 h of incubation with radioactively labelled [1,2-¹³C₂]glucose app. 20% of the labelled lactate was singly labelled demonstrating the significant contribution of the PPP and transketolase reaction to lactate production. In addition Braun et al. (Free Radic. Biol. Med. 23, 804-808, 1997) have demonstrated that transketolase enzyme reactions are involved in ascorbate metabolism leading to lactate. Ascorbate or dehydroascorbate is degraded via 5-carbon compounds and enters the PPP. In human erythrocytes and in MCF7 cells, ascorbate or dehydroascorbate caused a huge increase in lactate synthesis. Addition of oxythiamine inhibited lactate production accompanied with a decreased acorbate or dehydroascorbate consumption demonstrating the important role of the transketolase reaction for lactate production. TKTL1 expression and the Warburg effect

Warburg 80 years ago identified a metabolism characterized by a high glucose use leading to large amounts of lactate. He tried to answer the question 'what is the difference between fast growing embryo tissues and fast growing tumor tissues?' (Warburg et al., Biochem. Z. 152, 309-344, 1924). By analysing the metabolism of these tissues he recognized that both tissue types in the absence of oxygen degrade glucose to lactate. In the presence of oxygen Warburg detected a dramatic difference in metabolism. While the embryo tissue switched to the endoxidation metabolism, the tumor tissue still continued to degrade glucose to lactate and he already observed a correlation between the degree of anaerobic glucose degradation (aerobic glycolysis), lactate production and malignancy. Interestingly Warburg observed this type of anaerob metabolism also in healthy tissues like retina, spleen and testis. These tissues showed a mixture of anaerobic and aerobic glucose degradation. In those tissues where Warburg identified the aerobic glycolysis, a strong TKTL1 expression has been detected demonstrating a correlation of aerobic glycolysis and TKTL1 expression in healthy tissues.

### Lactate, hyaluronan, metastasis, and wound healing

An explanation for the high metastasis rate of lactate producing tumors has been suggested by Stern et al. (Exp. Cell. Res. 276, 24-31, 2002). Hyaluronan, a high-molecular-weight glycosamino-glycan of the extracellular matrix, is prominent during rapid tissue growth and repair. It stimulates cell motility and hydrates tissue, providing an environment that facilitates cell movement. Lactate production is used to recruit host fibroblasts to deposit hyaluronan and to express CD44, thereby participating in the process of cancer invasion and metastasis.

Lactate is also an important mediator of wound healing. It has been demonstrated to increase collagen production by cells of the tendon sheath, epitenon, and endotenon. Lactate accumulates to 4-12 mM in wounds.

### Diabetes and cancer

Tumors using hugh amounts of glucose and producing large amounts of lactate would benefit from an elevated blood glucose level. This explains why diabetes is an independent predictor of mortality from cancer of the colon, pancreas, female breast, and, in men, of the liver and bladder (Coughlin et al., Am. J. Epidemiol. 159, 1160-1167, 2004) and why patients with diabetes mellitus and high-risk stage II and stage III colon cancer experiences a significantly higher rate of overall mortality and cancer recurrence (Meyerhardt et al., J. Clin. Oncol. 21, 433-440, 2003).

### Threedimensional structure and molecular evolution of transketolases

All characterized transketolases have similar kinetic and physical properties, but the mammalian enzymes are more selective in substrate utilisation than the nonmammalian (Schenck et al., Int. J. Biochem. Cell Biol. 30, 1297-1318). The most prominent difference between the TKTL1 protein and known transketolase proteins is the deletion of an internal stretch of amino acid residues within the TKTL1 protein (amino acid residues homologous to yeast transketolase amino acid residues 68-107). The presence of mutated TKTL1 orthologues in mouse and rat demonstrate that the mutation leading to the deletion of the coding exon has occurred before the divergence of the human and murine lineage. Three transketolase genes could be detected in human and mouse, whereas until now only one transketolase gene has been detected in zebrafish (*Danio rerio*) and frog (*Xenopus laevis*). In human, mouse and rat three transketolase genes are present. Two transketolase genes harbor introns (TKT and TKTL1) whereas the TKTL2 gene represents an intronless gene. Based on the high homology to the TKTL1 gene and the intronless gene structure, the TKTL2 gene represents a reverse transcriptase mediated integration event of a former TKTL1 mRNA. At the timepoint of integration into the genome the TKTL1 gene was not mutated, since the TKTL2 does not have the internal deletion. The TKTL2 gene therefore represented a backup gene at the timepoint of insertion allowing the mutation of the TKTL1 gene without severe negative effects for the organism.

Since the TKTL1 and TKT proteins represent proteins well conserved with yeast transketolase, a protein of which the crystallographic structure is already determined and functional enzymatic analysis has been performed, it is important to put the information about the evolutionary changes of the TKTL1 protein in the context of what has been known about the transketolase enzyme reaction and the underlying threedimensional structure. Determination of the threedimensional structure of yeast transketolase led to the identification of invariant amino acid residues (HIS30, HIS263, ARG359, SER386, HIS469, ASP477, ARG528) which are building the substrate chanel. These amino acid residues are also present in the TKTL1 protein suggesting the presence of a transketolase substrate channel. The mutation in the TKTL1 gene led to the deletion of a histidin residue homologous to HIS103 of S.cerevisiae. The function of this invariant amino acid residue has been determined. Using site-directed mutagenesis H103 was replaced and the residual catalytic activities of the mutant enzymes were significantly lower concomitant with an increased Km values for the coenzyme thiamine. The Km values for the acceptor substrate R5P were similar to the Km value for wild-type transketolase, but the Km value for the donor substrate, X5P was increased more than tenfold in two mutants (Wikner et al., Eur. J. Biochem. 233, 750-755, 1995). Besides the substrate specifity the mutation also changed the reaction modus. Mutation of His103 in yeast transketolase led to a significant acceleration of an one-substrate reaction but a slow down of the two-substrate reaction so that the rates of both types of catalysis became equal (Selivanov et al., FEBS Lett. 567, 270-274, 2004). Furthermore Selivanov could demonstrate that the type of substrate reaction (one-substrate or two-substrate) and the affinity to thiamine is influenced in the HIS103 yeast transketolase mutant protein dependent on the presence of Ca(2+) or Mg(2+). This indicates that the presence of a mutated transketolase protein similar to the yeast HIS103 mutant would lead to an altered substrate spectrum, a reduced thiamine affinity and would allow a regulation by Ca(2+) or Mg(2+).

### One-substrate reaction of TKTL1 and convergence in evolution

The metabolic functions that were taken on by TKTL1 proteins in the course of evolution play a crucial role. Normal transketolases are thiamine dependent enzymes transferring a C2 unit from a ketose donor to an aldose acceptor. The normal transketolase catalysis two of such reactions within the non-oxidative pentose phosphate cycle. By use of the non-oxidative pentose phosphate cycle ribose (C5 sugar) can be converted into glucose (C6 sugar) and vice versa. By decomposing glucose via the pentose phosphate cycle to ribose reducing equivalents are generated as NADPH. This reaction provides the building blocks and precursors for DNA synthesis and, in addition, the reducing equivalents for processes of synthesis. In addition the TKTL1 protein is also able to perform enhanced backward reactions thereby allowing the building of metabolites e.g. C5-sugars from C3- and C2-units. Thus, the transketolase reaction has a central metabolic function.

The lower substrate specificity of the TKTL1 proteins being the result of the mutations allows the conversion of substrates that can not be used by other transketolases. Moreover, the TKTL1 proteins catalyse a more pronounced "one-substrate"-reaction whereas for normal transketolases (e.g. TKT) the "two-substrate"-reaction is favoured (normally resulting in the transfer of a C2 donor residue on a different acceptor). According to Bykova et al. (Biochem. Biophys. Res. Commun. 280, 845-847, 2001) an one-substrate reaction for the (unmutated) yeast transketolase has been described. Apart from the common two-substrate reaction with ketose as donor substrate and aldose as acceptor substrate, the yeast transketolase enzyme splits X5P into glyceraldehyde-3-phosphate and erythrulose. Erythrulose is formed by the reaction of free and enzyme-linked glycolaldehyde (Sevostyanova et al., Biochem. Biophys. Res. Commun. 313, 771-774, 2004). In yeast transeketolase this type of reaction is underrepresented and the normal two-substrate reaction is the main reaction. The mutations within the TKTL1 gene led to mutated TKTL1 protein isoforms with different substrate specificity and reaction modus compared to the human TKT protein. Some of the TKTL1 proteins are also capable of using a certain kind of sugar as a donor and as a acceptor. Thus, by use of this ketolase degradation pathway the cell can react on different metabolic situations in a very flexible manner. Using the recombinant as well as the human TKTL1 protein the inventors confirmed the formation of glyceraldehyd-3-phosphate in an one-substrate reaction utilizing X5P as sole carbon source. Depending upon the enzyme specificity of TKL1, the second product, formed by the one-substrate reaction, is of highest interest. An enzyme specialized to perform such an one-substrate reaction with X5P is present in heterofermentative lactic acid bacteria. This phosphoketolase (E.C. 4.1.2.9) belongs to the family of thiamine dependent enzymes and enables a metabolic pathway leading to lactate, and acetate or ethanol. Intermediate products of this metabolic pathway are acetylphosphate and acetyl-CoA. (Schlegel, Thieme Verlag, 1992). The metabolism of X5P via TKTL1 result in the formation of acetyl-CoA. The anaerobic glucose degradation under aerobic conditions leading to high amounts of lactate and the building of the energy rich compound acetyl-CoA. The pyruvate dehydrogenase complex is then inhibited by acetyl-CoA, and as a consequence, pyruvate is mainly reduced to lactate. This explains the Warburg effect, a phenomenon not understood since 80 years. The modified (trans)ketolase reaction of the TKTL1 is energetically superior to the normal transketolase reaction. The lactate building of the ketolase pathway, in contrast to the anaerob glycolysis (e.g. muscle) enables a sugar degradation without the use of oxygen, but does produce much more energy for a cell than the glycolytic pathway. The mutation in the TKTL1 proteins represent an example of evolutionary convergence, leading to a ketolase enzyme reaction already evolved early in history of life. This not yet described biochemical pathway is present in healthy tissues like retina, endothelial cells, nervous tissue and testicle and in tumor tissues. Tumors overexpressing the TKTL1 are producing large amounts of lactate leading to a matrix degradation. This facilitates tissue remodeling and wound healing. Therefore enhancing or reducing the activity of TKTL1 can be exploited to influence processes of tissue remodeling, wound healing etc.

Epithelial malignancies, cell cycle and moonlighting proteins The TKTL1 gene is located in Xq28, one of six chromosomal regions, which are both activated in malignancies and during cell cycle (Glinsky et al. Cancer Lett. 201, 67-77, 2003; Neoplasia 5, 218-228, 2003). The TKT and the TKTL2 gene are not located in such genomic regions. An activation of TKTL1 in S-phase is not surprising since there is a strong need for riboses as a basis for new DNA synthesis. The presence of TKTL1 protein within the cytoplasm is in agreement with such an expected enzymatic function. Additionally, nuclei of a subset of tumors do also show a staining. This staining appeared to be specific of cells in S or pre-mitotic cell cycle phases. This is very similar to the GAPDH protein. Although the GAPDH protein was well characterized as a glycolytic enzyme, it could be later shown that this protein executes additional functions, dependent from its localization. Besides diverse membrane and cytoplasmic functions, the nuclear function of GAPDH include also a role in apoptosis and neurodegenerative disorders (Berry, J. Psychiatry Neurosci. 29, 337-345, 2004).

A single protein with multiple, apparently unrelated, functions might seem surprising, but there are actually many cases of proteins that have more than one role in an organism. Proteins with multiple cellular functions depending on the subcellular localization, the cell type as well as its aggregation state are so called moonlighting proteins. Besides GAPDH, other evolutionary old metabolic enzymes such as aldolase (Wang et al., Exp. Cell. Res. 237, 445-451, 1997) or phosphoglucose isomerase/autocrine motility factor/neuroleukin/maturation factor (PGI/AMF/neuroleukin/MF) belong to this kind of proteins with multiple functions.

The moonlighting proteins GAPDH and TKTL1 share striking similarities. Both enzymes are evolutionary old proteins involved in sugar metabolism. Both proteins are mainly located in the cytoplasm, but also occur in the nucleus. GAPDH and TKTL1 have a function in cell cycle. Both proteins were found to be closely bound to each other in yeast (Kochetov et al., Biophys. Res. Commun. 40, 873-879, 1979) and the GAPDH protein and transketolase proteins/enzyme reactions have been associated with neurodegenerative diseases (Mazzola and Sirover, J. Neurosci. Methods. 137, 241-246, 2003; Gibson et al., Arch. Neurol. 45, 836-840, 1988; Paoletti et al., Biochem. Biophys. Res. Commun. 172, 396-401, 1990; Paoletti et al., J. Cell Physiol. 172, 63-68, 1997; Berry, J. Psychiatry Neurosci. 29, 337-345, 2004).

Protein interactions are important for the different functions of TKTL1 protein isoforms. TKTL1 protein isoforms are part of a multi-protein complex. Within this complex TKTL1 is also bound to transketolase unrelated proteins like glyceraldehyd-3-phosphate dehydrogenase (GAPDH), DNaseX (DNasel-like-1), Akt (= protein kinase B); histones, histon deacetylase, amyloid precursor protein and actin binding proteins.

During the experiments leading to the present invention different protein isoforms were identified on protein level using a novel monoclonal antibody (Linaris Biologische Produkte GmbH, Wertheim) specifically detecting the TKTL1 protein. By transcript and protein based analysis of the three TKT family members the present inventors were able to demonstrate that the TKTL1 gene is the transketolase gene/protein which is overexpressed in tumors. Known transketolases are homodimers of two full length proteins harbouring all typical invariant transketolase amino acid residues. The transketolase-like gene encoded TKTL1 protein isoforms build TKTL1 homo/heterodimers and TKT/TKTL1 heterodimers. The expression of TKTL1 protein isoforms - even an enzymatically non-active isoform - influences the enzymatic activity of a TKT protein as part of a TKT/TKTL1 heterodimer. A molecular switch and a proton wire synchronize the active sites in TKT/TKTL1 and TKTL1/TKTL1 homo- and heterodimers.

The proof that the TKTL1 gene (NM_012253; Accession Numbers: X91817; BC025382) encodes a mutated full length transketolase protein as well as smaller protein isoforms has important implications for basic research and medical health. Transketolase proteins and transketolase enzyme activities have been related to cancer, neurodegenerative diseases and diabetes and all the observed phenomenons have been interpreted solely on the basis of a single transketolase gene encoding a single protein. Up to now molecular or biochemical analysis failed to identify the TKT gene as the basis for these disease related phenomenons. A variant transketolase enzyme has been proposed to be associated with Wernicke-Korsakoff syndrome (Blass et al., 1977; Mukherjee et al., 1987). However, no mutations have been found so far in transketolase cloned from Wernicke-Korsakoff patients (McCool et al., 1993). In Alzheimer disease patients transketolase protein variants with different isoelectric points or a proteolytic cleavage leading to small transketolase protein isoforms have been identified (Paoletti et al., 1990; Paoletti et al., 1997). In diabetes mellitus patients the lipid-soluble thiamine derivative benfotiamine activates the TKT enzyme and blocks three major pathways of hyperglycemic damage and prevents diabetic retinopathy (Hammes et al., 2003).

Besides their enzymatic functions, these TKTL proteins exhibit various different functions depending on the localization of the proteins and their state of aggregation. As an example, within the cytoplasm the main function is the catalysis of the (trans-)ketolase reaction. Additional functions of the TKTL1 proteins located within the nucleus are associated with the control of the cell cycle and mitosis, control of transcription (the TKTL1 gene itself and others), and regulation of apoptosis. The TKTL1 proteins (with their functions depending on their localization or the state of aggregation) are designated as "moonlighting" proteins, since they execute different functions depending on subcellular localization, the cell type as well as its aggregation state.

Mutations of TKTL1 not only result in the lowering of substrate specificity but, in addition, in a lower affinity for thiamine. Thus, a reduced thiamine level leads to an increased failure of some of the TKTL1 proteins resulting in damages being due to decreased TKTL1 activity. These pathological changes can be avoided or at least corrected by activation of this metabolic pathway. Determination of thiamin affinity of TKTL1, or amount of TKTL1 or activity of TKTL1 can be expoited to identify individuals who should be treated with thiamine or thiamine-derivates which a better bioavailability (e.g. benfotiamine) to avoid or correct diabetes associated phenomenons like retinopathy, (cardiac autonomic) neuropathy, or damaging of endothelial cells.

Reduced TKTL1 enzyme activity can be identified in inviduals based on TKTL1 gene mutations, reduced or enhanced enzyme activities of the isolated TKTL1 protein or an in vivo imaging of the TKTL1 enzyme reaction. Reduced TKTL1 enzyme acitivities can be detected in still healthy individuals or in patients e.g. Alzheimer disease patients. Diagnosis can be performed by determining the enzymatic activity of the TKTL1 protein isolated from the patient (e.g. serum, liquor, or other body fluids) or by in vivo imaging of TKTL1 enzyme activity. Molecular imaging differs from conventional techniques because it identifies specific gene products and intracellular processes like specific enzyme reactions. The altered substrate specificity and reaction modus of the TKTL1 enzyme can be used for the detection of cells or tissues with an enhanced or reduced TKTL1 enzyme activity. The altered substrate specificity and reaction modus of the TKTL1 enzyme allows the discrimination between the enzymatic activity of three transketolase(-like) enzymes thereby allowing the measurement of TKTL1 enzymatic activity in vivo. The enzymatic activity can be detected by e.g. positron emission tomography, chemiluminiscence or radiographic imaging.

As used herein, chemiluminescence refers to a chemical reaction in which energy is specifically channeled to a molecule causing it to become electronically excited and subsequently to release a photon thereby emitting visible light. Temperature does not contribute to this channeled energy. Thus, chemiluminescence involves direct conversion of chemical energy to light energy. As used herein, luminescence refers to the detectable electromagnetic radiation, generally, UV, IR or visible electromagnetic radiation that is produced when the excited product of an exergonic chemical process reverts to its ground state with the emission of light. Chemiluminescence is luminescence that results from a chemical reaction. Bioluminescence is chemiluminescence that results from a chemical reaction using biological molecules (or synthetic versions or analogs thereof) as substrates and/or enzymes.

Kits containing the diagnostic systems and diagnostic systems that rely on bioluminescence for visualizing tissues in situ are provided. The systems include compositions containing substrates that are converted by the TKTL1 enzymatic activity. The systems can also include a composition that contains a bioluminescence generating reaction. Administration of the compositions results in production of light by targeted tissues that permits the detection and localization of cells or tissues e.g. for surgical removal.

Systems and methods for radiographic imaging of tissue using a radio-opaque imaging agent that in one embodiment accumulates intracellularly in tissue in proportion to its functional, or physiological, activity. In one embodiment, the imaging agent is a cell membrane-permeable, radio-opaque, selective substrate or high affinity ligand for TKTL1.

The present invention relates to ¹⁸F-labeled TKTL1 substrates and to methods of using same as imaging agents (for example, as positron emission tomography (PET) imaging agents) for the noninvasive detection and localization of cells and tissues with an enhanced or reduced TKTL1 enzymatic activity. The invention further relates to methods of synthesizing labeled substrates and to compositions comprising such analogs.

Patients with Wernicke-Korsakoff syndrome do harbor transketolases with a reduced affinity for thiamin. In Alzheimer's disease patients and in patients with Wernicke-Korsakoff syndrome, transketolase protein variants with different isoelectric points or smaller size have been observed. In diabetes patients the lipid-soluble thiamine derivative benfotiamine blocks three major pathways of hyperglycemic damage and prevents diabetic retinopathy (Hammes et al., Nat. Med. 9, 294-299, 2003). The positive results of a benfotiamine treatment has been interpreted as a consequence of activation of the TKT protein. The inventors were able to identify TKTL1 proteins in those tissues (endothelial cells, retina, and nerves) in which patients with diabetes get cell damage due to AGE (advanced glycation end-product) formation or cell death.

### Insulin, diabetes, obesity and cancer

Type 2 diabetes continues to increase in prevalence and mirror the rising epidemic of obesity. In particular, type 2 diabetes is becoming more common in children and adolescents. Targeting patients at risk for diabetes as well as the aggressive management of patients diagnosed with type 2 diabetes are key to preventing the development of comorbid conditions that negatively impact patient health and increase associated health care costs. Disturbances of glucose metabolism including impaired glucose tolerance and both insulin-dependent and non-insulin-dependent diabetes causes neurodegeneration. An increasing number of degenerative diseases of neuronal tissues are known to be associated with diabetes mellitus, increased insulin resistance and obesity, disturbed insulin sensitivity, and excessive or impaired insulin secretion. These syndromes include Alzheimer disease, ataxia-telangiectasia, Down syndrome/trisomy 21, Friedreich ataxia, Huntington disease, several disorders of mitochondria, myotonic dystrophy, Parkinson disease, Prader-Willi syndrome, Werner syndrome, Wolfram syndrome, mitochondrial disorders affecting oxidative phosphorylation, and vitamin B(1) deficiency/inherited thiamine-responsive megaloblastic anemia syndrome.

When blood sugar levels rise, some key kinds of cell - including nerve cells (neurons) and the cells that make up the fine blood cells of the retina of the eye and the filtering units (glomeruli) of the kidney - are also flooded with glucose. The resulting high sugar levels within these cells cause a logjam in the normal cellular metabolism of glucose. This backlog results in a buildup within the cell of super-reactive glucose-metabolic intermediates known as triosephosphates. And once that happens, the excess triosephosphates attack the surrounding proteins, lipids, and DNA, causing AGE damage from within the heart of the cell. It's these cells are thus the most vulnerable to the complications of diabetes and other functional disorders. Western diets, which are frequently poor in thiamine and high in sugars, result in increased levels of endogenous glyoxals, which in turn lead to a predisposition to AGE (advanced glycation end-product)-related pathologies and neoplastic conditions. AGE form by a nonenzymatic reaction between reducing sugars and biological proteins. These stable compounds accumulate slowly throughout the life span and contribute to structural and physiologic changes in the cardiovascular system such as increased vascular and myocardial stiffness, endothelial dysfunction, altered vascular injury responses, and atherosclerotic plaque formation. Mechanisms underlying these alterations include AGE cross-linking of collagen and AGE interactions with circulating proteins and AGE receptors. Glyoxals are involved in this processes. Glyoxals are reactive alpha-oxoaldehydes that are formed endogenously from sugars, the levels of which are increased in various pathological conditions associated with hyperglycaemia and thiamine deficiency. The clinical manifestations of AGE accrual-isolated systolic hypertension, endothelial and diastolic dysfunction, and atherosclerosis-underscore their role in increased cardiovascular risk associated with aging as well as diabetes and hypertension, conditions that enhance AGE formation. AGE formation contribute to the progression of atherosclerosis, particularly in diabetes.

New pharmacologic agents based on influencing the TKTL1 proteins are the basis for the prevention of AGE formation, inhibition of protein cross-links, reduction of vascular and myocardial stiffness, inhibition of atherosclerotic plaque formation, and improvement in endothelial function. These agents reduce the risk of isolated systolic hypertension, diastolic dysfunction, diabetes, and heart failure. Therefore, it is one object of the invention to identify agents for the prevention of AGE formation by influencing TKTL1 proteins.

Glycation is also involved in amyloidogenesis and protein plaque generation leading to neurodegenerative diseases. The fibrils present in Alzheimer disease (AD) patients share several properties common to glycated proteins. Glycation causes the structural transition from folded, soluble form to beta-fibrils. Inhibition of glycation by activation of the TKTL1 enzyme activity can be performed (by e.g. thiamin application in food) to prevent the building of fibrils and therefore the onset of neurodegenerative diseases in individuals with a reduced TKTL1 activity.

Impaired brain sugar metabolism essentially always occurs in clinically significant AD, and the degree of clinical disability is proportional to the degree of metabolic impairment. The earliest, mildest changes in brain metabolism occur even before the onset of measurable cognitive impairment or atrophy. The TKTL1 enzyme activity is the limiting step in glucose metabolism via PPP. Enhancing the TKTL1 activity can prevent the development of AD in individuals with a predisposition due to TKTL1. Thus, in a further embodiment of the invention a method to determine individuals with TKTL1 such predisposing variants to identifiy individuals eligible for a preventive TKTL1 therapy is provided.

### Brief description of the drawings

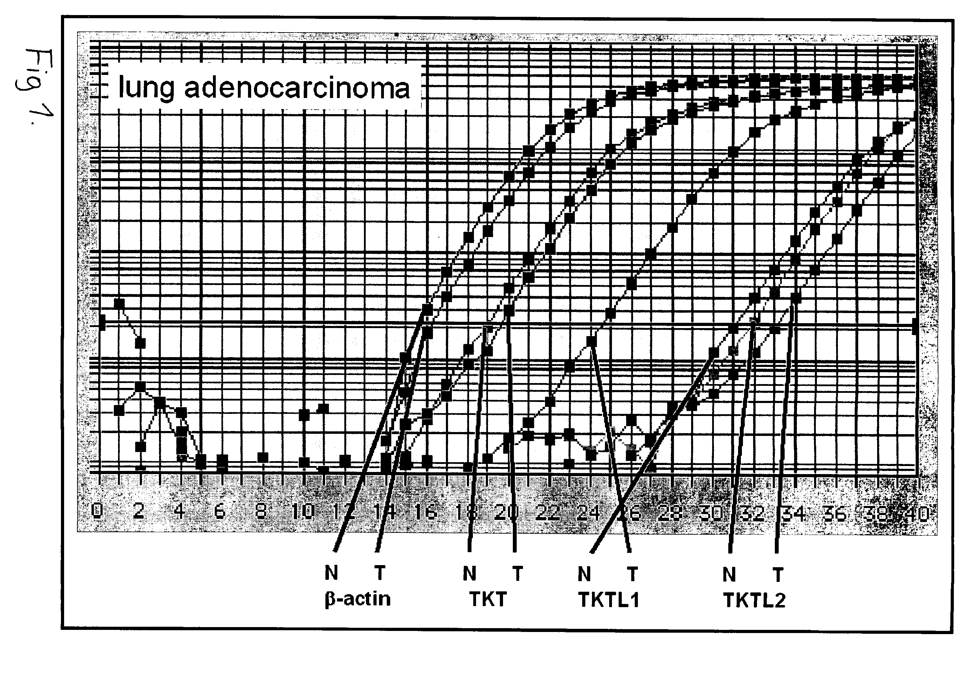

Figure 1: (A) Quantification of TKTL1 transcripts in gastric and lung adenocarcinoma samples and their corresponding normal tissues. N - normal sample; T - tumor sample; M - marker, 100 bp and 200 bp fragments are shown. 15 µl of the real-time PCR reaction was loaded onto a 3%-agarose gel to visualize the 150 bp TKTL1 amplification product. Expression differences between tumor and corresponding normal tissue have been calculated on basis of the real-time data and are shown as fold-induction in tumor sample relative to the corresponding normal sample. (B) Real-time transcript quantification of TKT, TKTL1, TKTL2, and β-actin gene in a lung adenocarcinoma and corresponding normal sample. The highest expression level is observed for the β-actin. Within the transketolase gene family, the TKT gene shows the highest expression level. The TKTL1 and TKTL2 expression level in normal lung is low compared to that of TKT and β-actin. In contrast to this, the expression level of TKTL1 in lung adenocarcinoma is 60-fold higher than in the corresponding normal tissue.

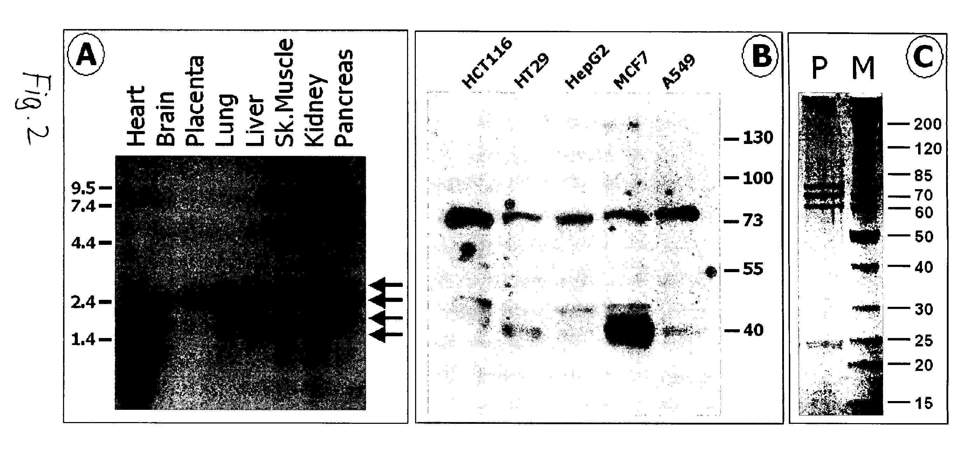

Figure 2: (A) Expression pattern of the human TKTL1 gene on northern blots of poly(A)⁺mRNA from different human adult tissues analysed with a TKTL1 cDNA probe. Four transcripts of 1.4, 1.9, 2.5, and 2.7 kb are detectable. Whereas the main transcript in most tissues is 2.5 kb in size, in heart the small transcript of 1.4 is abundant and the 2.5 and 2.7 kb transcripts are missing. Transcript sizes are indicated in kb. (B) Expression of TKTL1 protein isoforms in five tumor cell lines derived from four different tumor entities. Proteins were detected using a MAb specifically detecting TKTL1 protein isoforms and not reacting with other transketolase family members. Each cell line do show a unique expression pattern of TKTL1 protein isoforms. The molecular weight standard is indicated in kDa. (C) The TKTL1 full length protein was expressed in *E*. *coli* and was isolated by affinity purification through the N-terminal His-tag. One µg of affinity purified TKTL1 protein was loaded onto a 4-20%-gradient SDS gel and stained with Coomassie. Proteins different in size were detected. The largest protein (66kDa) represents the N-terminal His-tagged full length TKTL1 protein, whereas smaller TKTL1 proteins are likely due to C-terminal proteolytic cleavage already present prior to isolation procedure. Note that the migration of the recombinant 66kDa His-tagged TKTL1-full length protein indicates a size of 75 kDa. Sizes of the protein marker are indicated in kDa.

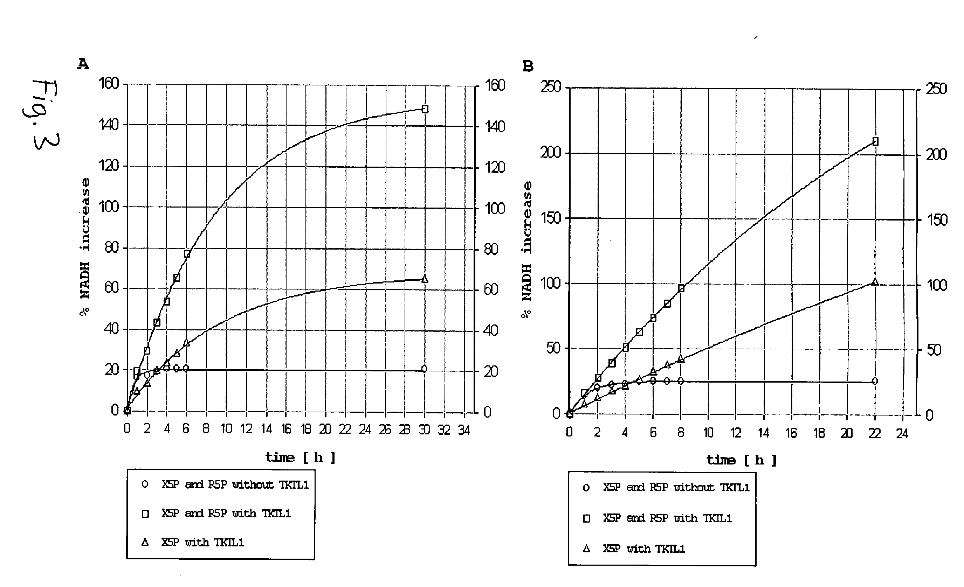

Figure 3: Determination of transketolase activity of native (A) and recombinant (B) TKTL1 protein. Two-substrate and one-substrate reaction was determined by the production of NADH as measured by gain in absorbance at 340 nm. Xylulose-5-phosphate (X5P) and ribose-5-phosphate (R5P) were used to determine the two-substrate reaction, whereas X5P alone was used for the one-substrate reaction. One representative of three independent enzymatic assays leading to similar results is shown.

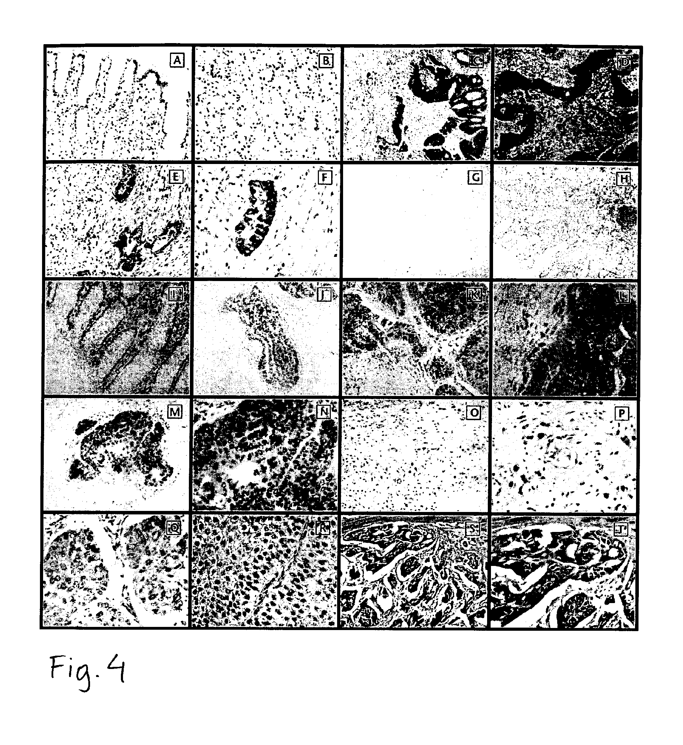

Figure 4: A-N: TKTL1 protein expression in tumors of three gastric carcinoma patients (A-P), a colon carcinoma patient (Q), and noninvasive (R) and invasive (S-T) bladder carcinoma patients. A-F: Specimens of gastric carcinoma patient 1 and corresponding normal tissue; G-N: Specimens of gastric carcinoma patient 2 and corresponding normal tissue. O,P: Specimens of gastric carcinoma patient 3. A,B: No expression of TKTL1 in normal corpus tissue. C-F: Strong cytoplasmic expression in tumor tissue, no expression in the surrounding stroma cells. Note the heterogenous expression in tumor cells (E,F). G-I: No expression of TKTL1 in normal antrum tissue. J-N: Strong cytoplasmic expression within tumor cells. Heterogenous expression in tumor cells (L). O,P: Nuclear expression in a poorly differentiated gastric carcinoma. Q: Cytoplasmic staining in a colon carcinoma. R: No expression of TKTL1 in a superficial bladder carcinoma. S,T: Strong cytoplasmic expression in an invasive poorly differentiated bladder carcinoma. Anti-TKTL1 was revealed by diaminobenzidine tetrahydrochloride (DAB; brown staining). Counterstaining was performed with haematoxylin (blue staining). Magnification: G x50; C,H, and J x100; A,B,D,E,I,K,M,O, and S x200; F,L,N,P,Q,R and T x400.

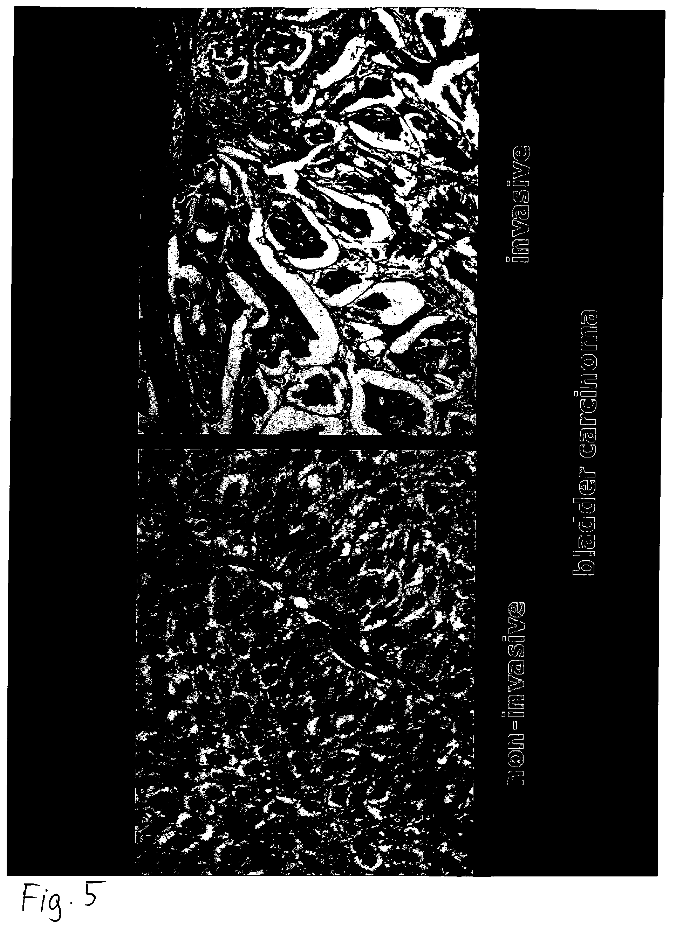

Figure 5: TKTL1 staining of noninvasive and invasive bladder carcinoma.

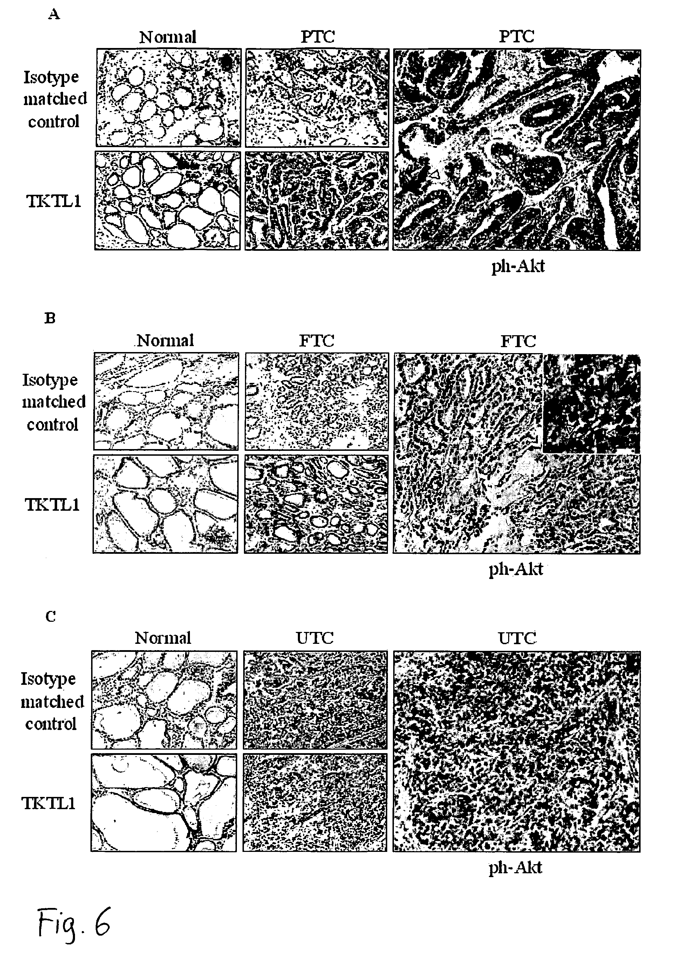

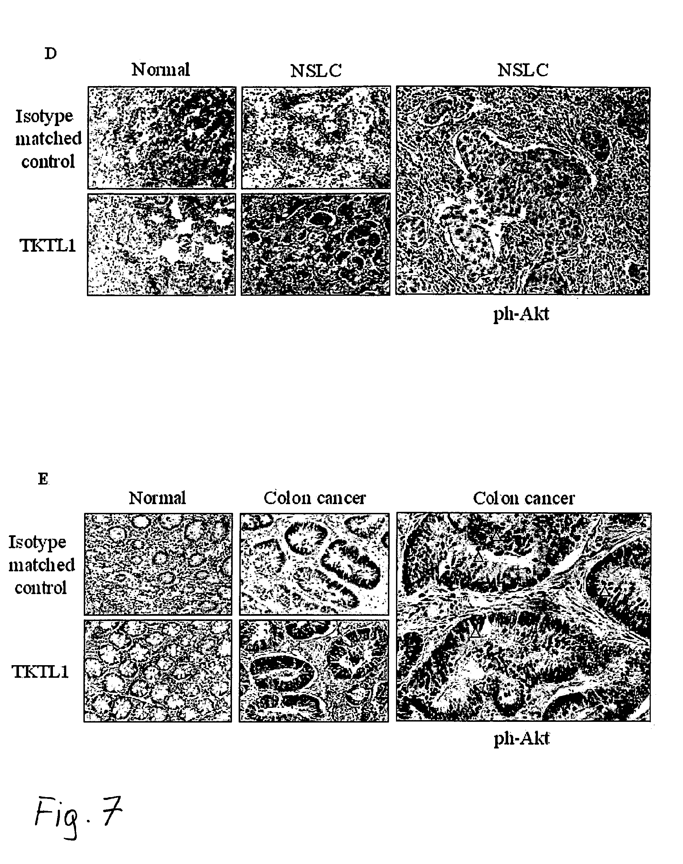

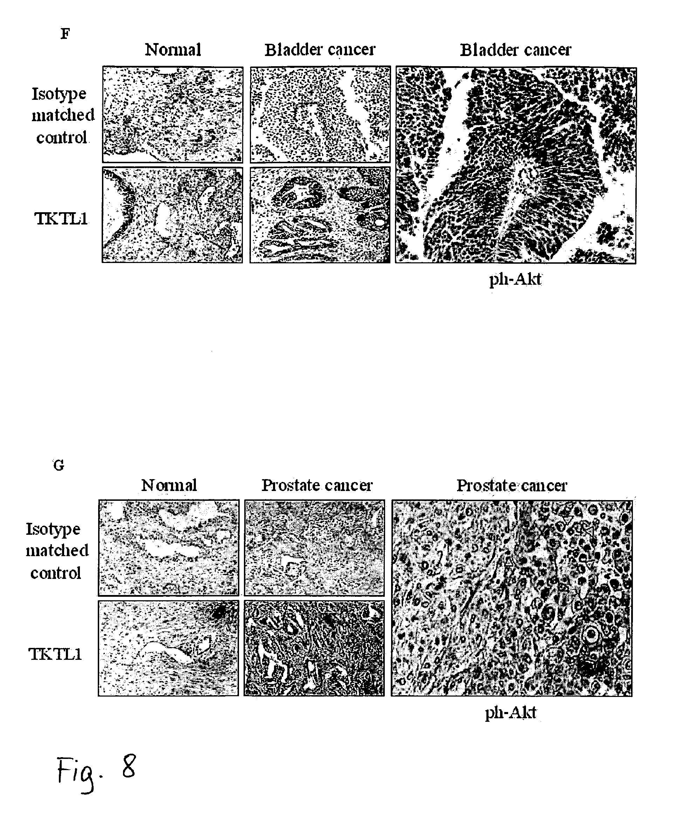

Figure 6-8: Expression of TKTL1 and phosphorylated Akt (ph-Akt) in cancer and normal tissue. Anti-TKTL1 or anti-ph-Akt was revealed by 3-amino-9-ethylcarbazole (AEC; red staining). Counterstaining was performed with haematoxylin (blue staining). Negative controls were performed using isotype matched IgG. One representative of three independent experiments is shown. Yellow arrowheads indicate nuclear ph-AKT staining. A-C: TKTL1 and phosphorylated Akt are highly expressed in thyroid cancer tissue. Immunohistochemical analysis on paraffin-embedded sections of normal, papillary (PTC), follicular (FTC), and undifferentiated (UTC) thyroid cancer labelled with anti-TKTL1 or anti-ph-Akt. D,E: Non small lung cancer (NSLC) and colon carcinomas express high levels of TKTL1 and phosphorylated Akt. (D), normal and NSLC tissues were stained with anti-TKTL1 or ph-Akt. (E), colon cancer specimens were immunoreactive to TKTL-1 or ph-Akt. F,G: Expression of TKTL1 and phosphorylated Akt in bladder and prostate carcinomas. Immunohistochemical analysis of TKTL1 or ph-Akt on paraffin sections of normal or bladder (F) and prostate (G) cancer specimens.

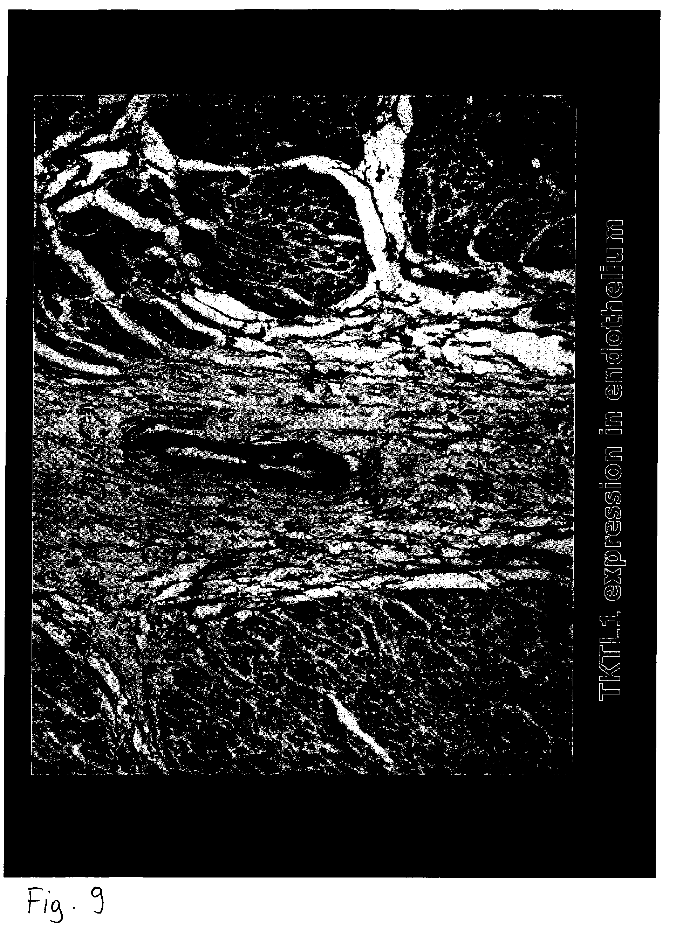

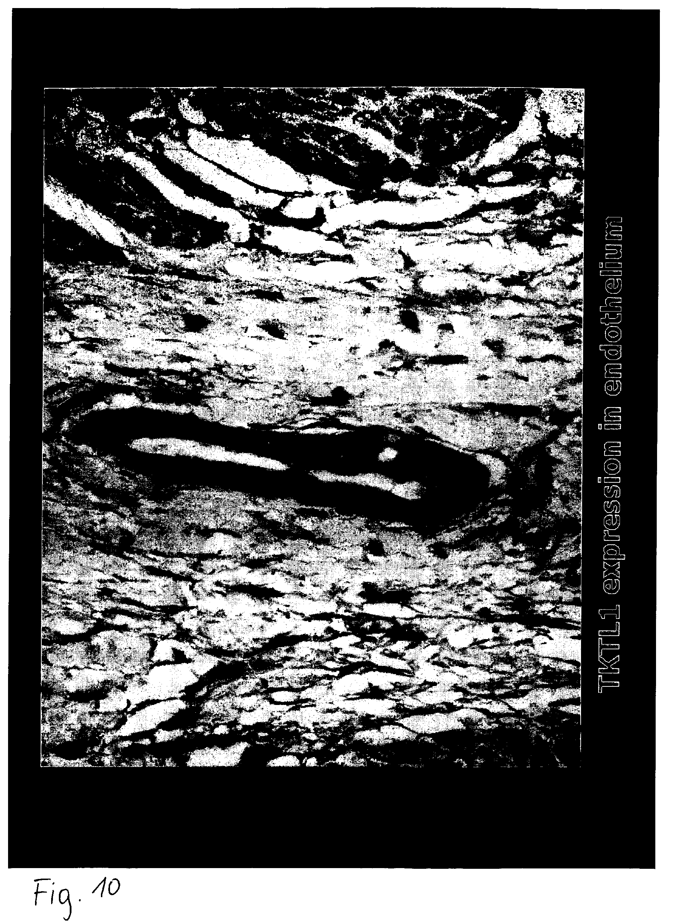

Figure 9-10: Expression of TKTL1 in endothelial cells.

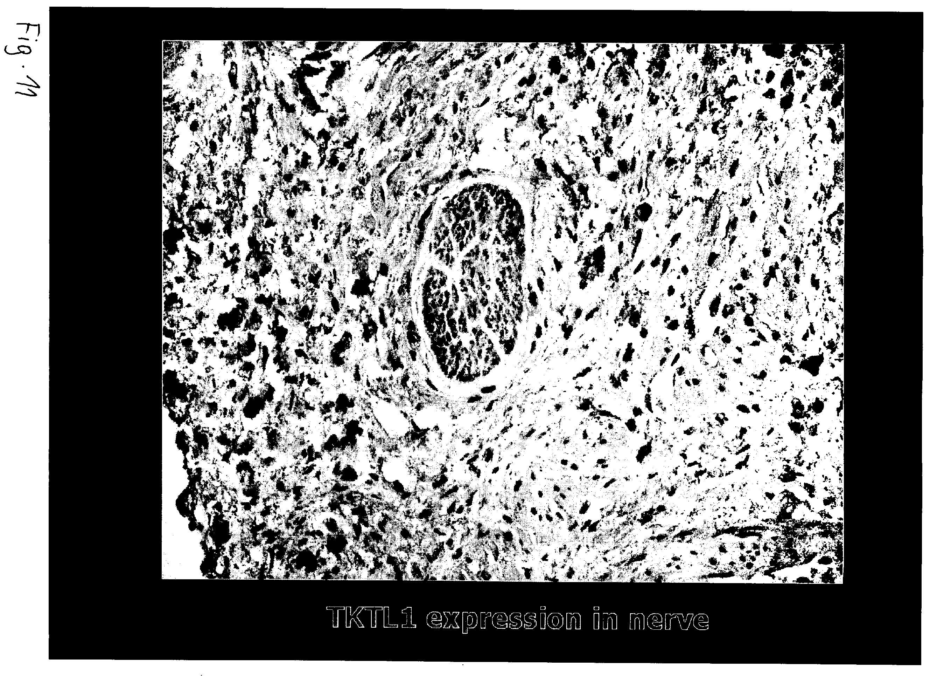

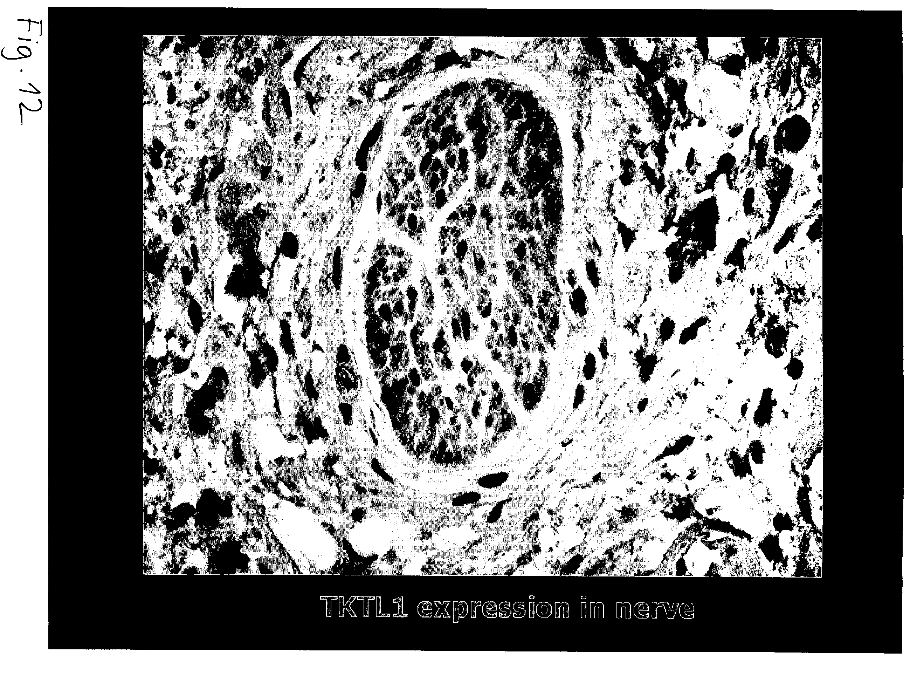

Figure 11-12: Expression of TKTL1 in neuronal cells.

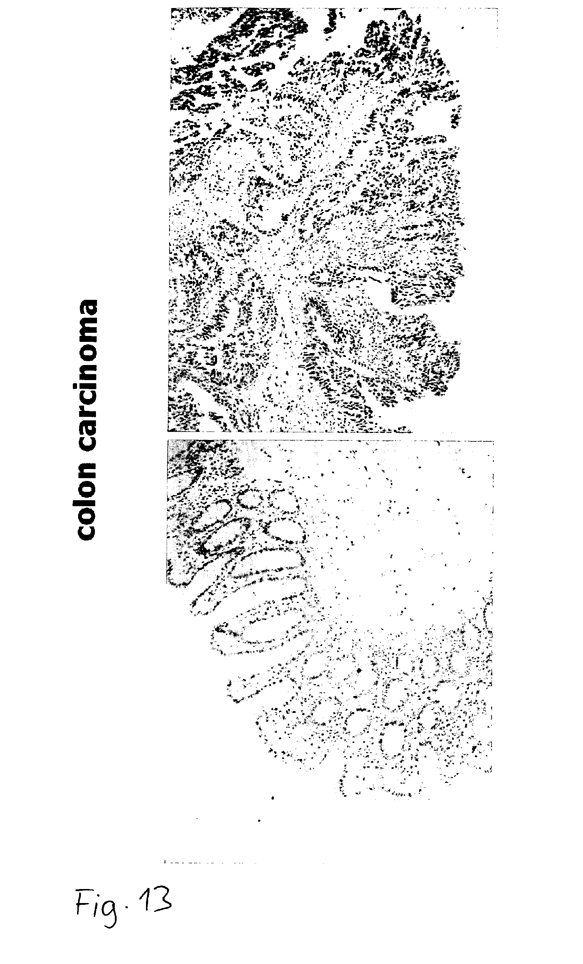

Figure 13: Expression of DNaseX in colon carcinoma and corresponding normal tissue.

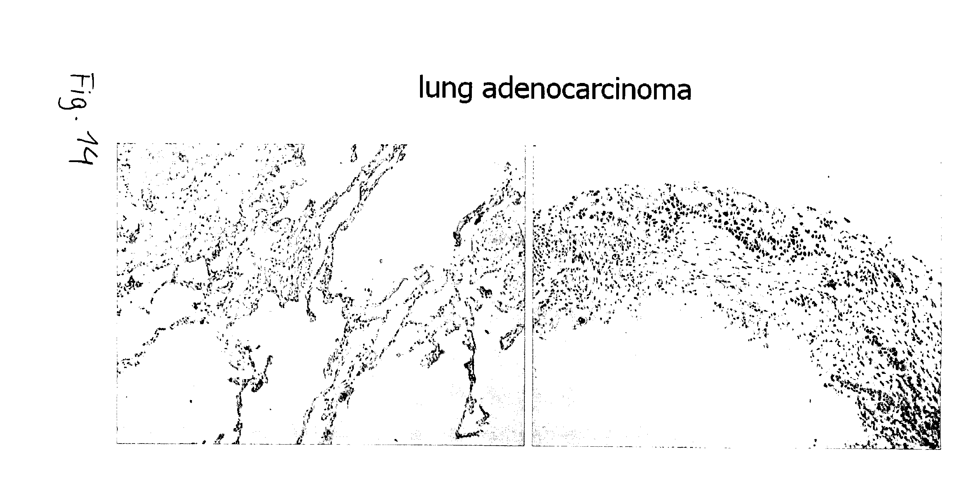

Figure 14: Expression of DNaseX in lung carcinoma and corresponding normal tissue.

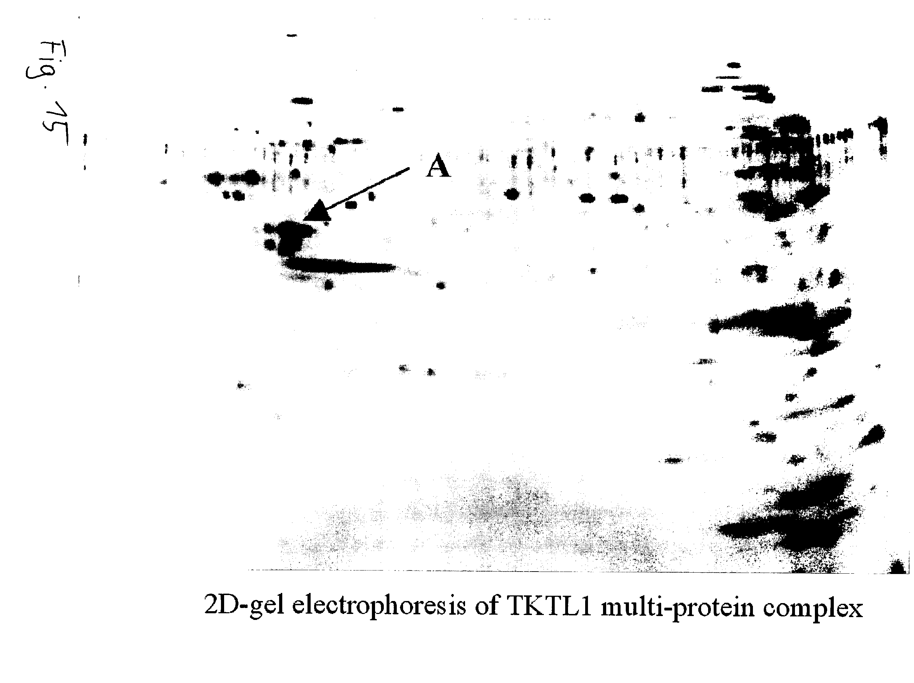

Figure 15: 2-dimensional (2D)-gel electrophoresis of a multi-protein complex harboring TKTL1, DNaseX, and GAPDH. The multi-protein complex was affinity-purified from human chronic myelogenous leukemia K562 cells using TKTL1 antibody JFC12T10 coupled to carbo-link. TKTL1 protein isoforms (arrow A) and other proteins present in the complex were identified by immunostaining and sequence determination.

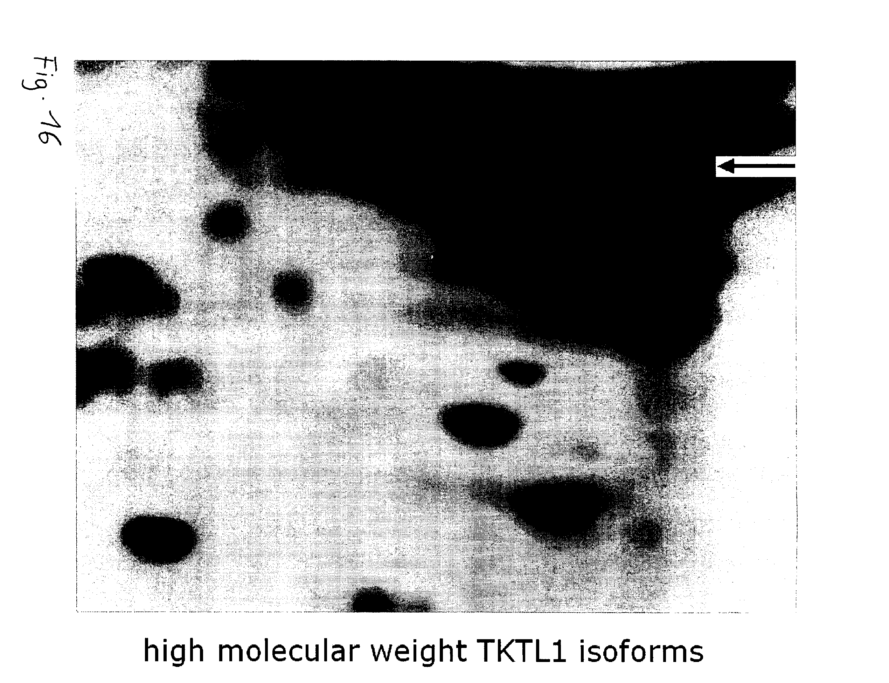

Figure 16: Identification of high molecular weight TKTL1 protein isoforms from a patient with a neurodegenerative disease (AD) by 2D-gel electrophoresis (arrow). TKTL1 protein isoforms were identified by immunostaining.

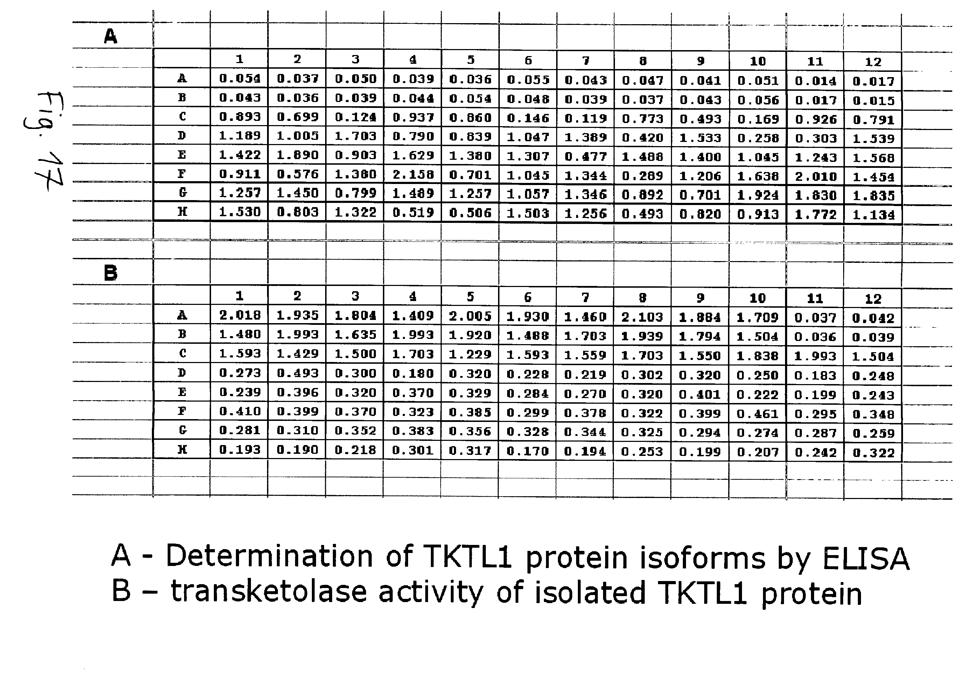

Figure 17: (A) Determination of TKTL1 protein isoforms by ELISA. The combination of TKTL1 antibody JFC6T8 and JFC5T3 determines a TKTL1 protein isoform specifically present in patients with neurodegenerative diseases. A1-A5 represent ELISA values obtained from leukocytes from healthy persons. A6-A10 represent ELISA values obtained from fibroblasts from healthy persons. B1-B5 represent ELISA values obtained from serum from healthy persons. B6-B10 represent ELISA values obtained from brain cells from healthy persons. A11-A12 and B11-B12 represent values obtained without probe material (background level).
   C1-C3 represent results obtained from leukocytes from AD patients.
   C4-C6 represent results obtained from fibroblast from AD patients.
   C7-C9 represent results obtained from serum from AD patients.
   C10-C12 represent results obtained from brain cells from AD patients.
   D1-D3 represent ELISA results obtained from leukocytes from Morbus Parkinson patients.
   D4-D6 represent ELISA results obtained from fibroblast from Morbus Parkinson patients.
   D7-D9 represent ELISA results obtained from serum from Morbus Parkinson patients.
   D10-D12 represent ELISA results obtained from brain cells from Morbus Parkinson patients.
   E1-E3 represent ELISA results obtained from leukocytes from Huntington disease patients.
   E4-E6 represent ELISA results obtained from fibroblast from Huntington disease patients.
   E7-E9 represent ELISA results obtained from serum from Huntington disease patients.
   E10-E12 represent ELISA results obtained from brain cells from Huntington disease patients.
   F1-F3 represent ELISA results obtained from leukocytes from SLE patients.
   F4-F6 represent ELISA results obtained from fibroblast from SLE patients.
   F7-F9 represent ELISA results obtained from serum from SLE patients.
   F10-F12 represent ELISA results obtained from kidney cells from SLE disease patients.
   G1-G3 represent ELISA results obtained from leukocytes from Morbus Parkinson patients.
   G4-G6 represent ELISA results obtained from fibroblast from Morbus Parkinson patients.
   G7-G9 represent ELISA results obtained from serum from Morbus Parkinson patients.
   G10-G12 represent ELISA results obtained from kidney cells from Morbus Parkinson disease patients.
   H1-H3 represent ELISA results obtained from leukocytes from diabetes type II Morbus Parkinson patients.
   H4-H6 represent ELISA results obtained from fibroblast from diabetes type II patients.
   H7-H9 represent ELISA results obtained from serum from diabetes type II patients.
   H10-H12 represent ELISA results obtained from kidney cells from diabetes type II disease patients.
   (B) Determination of enzymatic activity of TKTL1 isolated from healthy and patient derived specimens. TKTL1 antibody JFC3T9 was coupled to an ELISA plate, and incubated with samples from healthy persons and patients. After removing unspecific bound material, enzymatic activity was determined as described above. High enzymatic activities were obtained in samples of healthy persons (A1-A10, B1-B10, C1-C12). A1-A5 represent enzymatic activities obtained from leukocytes from healthy persons. A6-A10 represent enzymatic activities obtained from fibroblasts from healthy persons. B1-B5 represent enzymatic activities obtained from serum from healthy persons. B6-B10 represent enzymatic activities obtained from brain cells from healthy persons. A11-A12 and B11-B12 represent values obtained without probe material (background level).
   C1-C3 represent enzymatic activities obtained from endothelial cells from healthy persons.
   C4-C6 represent enzymatic activities obtained from neuronal cells from healthy persons.
   C7-C9 represent enzymatic activities obtained from kidney cells from healthy persons.
   C10-C12 represent enzymatic activities obtained from colon cells from healthy persons.
   D1-D3 represent enzymatic activity obtained from leukocytes from AD patients.
   D4-D6 represent enzymatic activity obtained from fibroblast from AD patients.
   D7-D9 represent enzymatic activity obtained from serum from AD patients.
   D10-D12 represent enzymatic activity obtained from brain cells from AD patients.
   E1-E3 represent enzymatic activity obtained from leukocytes from Morbus Parkinson patients.
   E4-E6 represent enzymatic activity obtained from fibroblast from Morbus Parkinson patients.
   E7-E9 represent enzymatic activity obtained from serum from Morbus Parkinson patients.
   E10-E12 represent enzymatic activity obtained from brain cells from Morbus Parkinson patients.
   F1-F3 represent enzymatic activity obtained from leukocytes from SLE patients.
   F4-F6 represent enzymatic activity obtained from fibroblast from SLE patients.
   F7-F9 represent enzymatic activity obtained from serum from SLE patients.
   F10-F12 represent enzymatic activity obtained from kidney cells from SLE disease patients.
   G1-G3 represent enzymatic activity obtained from leukocytes from multipe sclerosis patients.
   G4-G6 represent enzymatic activity obtained from fibroblast from multipe sclerosis patients.
   G7-G9 represent enzymatic activity obtained from serum from multipe sclerosis patients.
   G10-G12 represent enzymatic activity obtained from kidney cells from multipe sclerosis patients.
   H1-H3 represent enzymatic activity obtained from leukocytes from diabetes type II diabetes type II patients.
   H4-H6 represent enzymatic activity obtained from fibroblast from diabetes type II patients.
   H7-H9 represent enzymatic activity obtained from serum from diabetes type II patients.
   H10-H12 represent enzymatic activity obtained from kidney cells from diabetes type II disease patients.

### Immunohistochemical stainings shown in Figure 6,7,8 were performed as follows:

Five µm thick human cancer and normal paraffin sections were analyzed by immunohistochemistry. Dewaxed sections were heated for antigen unmasking in 10 mM sodium citrate (pH 6.0) for 1 minute at 450 W followed by 5 minutes at 100 W. After rinse in dH₂O, inhibition of endogenous peroxidase was performed with 5 min incubation with 3%-H₂O₂. Then, sections were exposed to biotin blocking system (DAKO) for 10 min to block endogenous avidin-biotin. After two washes in Tris/saline buffer (TBS), slides were incubated with 1% goat serum for 30 min to block unspecific staining. Successively, sections were exposed to mouse anti-TKTL1 (clone JFC12T10; mouse IgG2_{b}) antibody (25 µg/ml) or anti- Ser473 phospho-Akt (587F11; mouse IgG2_{b}; Cell Signaling Technology) overnight at 4°C. Then slides were washed in TBS and incubated with biotinilated anti-mouse immunoglobulins for 30 min at room temperature and treated with streptavidin-peroxidase (DAKO). Staining was revealed using 3-amino-9-ethylcarbazole (AEC) substrate. Nuclei counterstaining was performed using aqueous haematoxylin.

The immunohistochemical stainings shown in Figure 4,5, and 9-16 were performed as follows:

Three µm thick paraffin sections were heated for antigen unmasking in 10 mM sodium citrate (pH 6.0) for 5 minutes at 900 W, for 5 min at 900 W in dH₂0, and for 5 min in 10 mM sodium citrate (pH 6.0) at 900 W. After a wash in phosphate/saline buffer (PBS), inhibition of endogenous peroxidase was performed as above discribed. Then, sections were exposed 15 min to biotin-avidin blocking buffer (Vector Laboratories). Blocking of unspecific staining was performed with goat serum as described above. Primary antibodies were visualized with avidin-biotinylated horseradish peroxidase complex (ABC) and diaminobenzidine tetrahydrochloride (DAB) (Elite kit; Vector Laboratories), and counterstained with Mayer's haematoxylin.

### Tumor Cell Lines and Western Blot

The lung carcinoma cell line A549, the breast carcinoma cell line MCF7, the liver carcinoma cell line HepG2, and the colon carcinoma cell lines HCT116 and HT29 were obtained from ATCC. Cells were grown in RPMI 1640 or DMEM supplemented with 10% FCS, penicillin and streptomycin (Invitrogen) at 37°C with 5% CO₂.

### Northern blot analysis

A DNA probe from the 3' untranslated region (residue 1627 to 2368) of the TKTL1 transcript (acc. no. X91817) was labelled with [[alpha]-³²P]DATP and [[alpha]-³² P]dCTP (3000 Ci/mmol) in a random primed reaction (Feinberg and Vogelstein, 1983). Hybridization was carried out in 0.5 M sodium phosphate, 7% SDS, 0.2% bovine serum albumin, 0.2% PEG 6000, 0.05% polyvinylpyrrolidone 360000, 0.05% Ficoll 70000 and 0.5% dextran sulphate at 65°C overnight. Non-specifically bound probe was removed by washing at 65°C in 40 mM sodium phosphate, pH 7.2, 1% SDS for 60 min. Filters were exposed to X-ray film (Kodak) for 1-5 days. A multiple human adult tissue poly(A)⁺ RNA northern blot was purchased from BD Biosciences Clontech.

### Western blot analysis

For Western blot analysis, cells were lysed in lysis buffer (50 mM Tris-HCl pH 7.5, 150 mM sodium chloride, 1% NP40, 0.5% sodium deoxycholate, 0.1% SDS, 0.02% sodium azide, 1 mM phenylmethylsulfonyl fluoride). Aliquots of 50 µg of soluble protein was loaded into each well, electrophoresed on 12.5% SDS-polyacrylamide gels, and transferred to polyvinylidene difluoride membranes (Millipore). For detection of TKTL1-proteins the HRP-coupled JFC12T10 MAb was used in a final concentration of 1 µg/ml. The MAb was visualized with an ECL Western blot detection system (Amersham Pharmacia Biotech).

### Enzyme-linked immunosorbent assay (ELISA)

TKTL1 protein affinitiy-purified from cell lines was determined using common standard ELISA techniques. Three different affinity-purified mouse IgG monoclonal anti-TKTL1 antibodies (5 µg/ml) were used for coating of ELISA plates. Horseradish peroxidase conjugated anti-TKTL1 antibody JFC12T10 was used at 5 µg/ml as the secondary reagent. Bound proteins in the multi-protein complex were affinitiy-purified from cell lines using antibodies directed TKTL1, DNaseX, ph-Akt, GAPDH. Binding to certain proteins was assed by ELISA technique, using e.g. the combination of TKTL1 and GAPDH antibodies; and the combination of TKTL1 and DNaseX antibodies; and the combination of ph-Akt and DNaseX antibodies.

### 2D-analysis of multi-protein complexes

The samples were analysed by high resolution 2D gel electrophoresis (8x7 cm). 2.5 µg of protein was applied to two 2D gels for each sample and 2D gel was stained by silver and the proteins of the second one were transferred to PVDF membranes by semidry electroblotting for immunostaining.

Accordingly, in a first aspect, the present invention relates to a method for the diagnosis of a disease associated with a dysfunction of a TKTL1 protein in a biological sample obtained from a patient, wherein said method comprises determining the activity or level of the TKTL1 protein, the cellular localization of the TKTL1 protein, its aggregation status and/or its dimerization status, wherein an enhanced or decreased level of activity, an abnormal cellular localization of the mutated TKTL1 protein, aggregation status and/or dimerization status compared to a control is indicative of such disease.

TKTL1 gene products as used in the context of the present invention may comprise polypeptides and nucleic acids encoded by the transketolase like-1 gene. The polypeptides and polynucleotides (cf. TKTL1, TKR: NM_012253; Accession numbers: X91817; BC025382) used for performing the method according to the present invention are isolated. This means that the molecules are removed from their original environment. Naturally occurring proteins are isolated if they are separated from some or all of the materials, which coexist in the natural environment. Polynucleotides are isolated for example if they are cloned into vectors.

TKTL1 nucleic acid molecules used for performing (as a probe) a diagnostic method according to the present invention may comprise polynucleotides or fragments thereof. Preferred polynucleotides may comprise at least 20 consecutive nucleotides, preferably at least 30 consecutive nucleotides and more preferably at least 45 consecutive nucleotides, that are identical, share sequence homology or encode for identical, or homologous polypeptides, compared to the wild type transketolase like-1 polypeptides, but do not encode other transketolase like polypeptides or transketolases. The nucleic acids according to the present invention may also be complementary or reverse complementary to any of said polynucleotides. Polynucleotides may for example include single-stranded (sense or antisense) or double-stranded molecules, and may be DNA (genomic, cDNA or synthetic) or RNA. RNA molecules comprise as well hnRNA (containing introns) as mRNA (not containing introns).

According to the present invention the polynucleotides may also be linked to any other molecules, such as support materials or detection marker molecules, and may, but need not, contain additional coding or non-coding sequences.

The TKTL1 polynucleotides used according to the present invention may be native sequences or variants thereof. The variants may contain one or more substitutions, additions, deletions and/or insertions such that the immunogenicity of the encoded polypeptide is not diminished. Variants may for example be allelic variations of the polynucleotides. Allelic variation as used herein is an alternative form of the gene, which may result from at least one mutation in the nucleic acid sequence. Alleles may result in altered mRNAs or polypeptides whose structure or function may or may not be altered. Any given gene may have none, one, or many allelic forms. Common mutational changes, which give rise to alleles, are generally ascribed to natural deletions, additions, or substitutions of nucleotides. Each of these types of changes may occur alone or in combination with the others, one or more times in a given sequence. The variants according to the present invention show preferably 70%, more preferably at least 80% and most preferably at least 90% of sequence identity to the native nucleic acid molecules disclosed herein. Methods for determination of sequence similarity are known to those of ordinary skill in the art.

One example for detecting the similarity of sequences may be carried out using the FastA and/or BlastN bioinformatics software accessible on the HUSAR server of the DKFZ Heidelberg.

Nucleic acids as used in the context of the present invention may be all polynucleotides, which hybridise to probes specific for the transketolase like-1 sequences used herein under stringent conditions. Stringent conditions applied for the hybridisation reaction are known to those of ordinary skill in the art and may be applied as described in Sambrook et al. Molecular cloning: A Laboratory Manual, 2nd Edition, 1989.

The TKTL1 nucleotide sequences used according to the present invention may be joined to a variety of other nucleic acid sequences using the known recombinant DNA techniques. The sequences may for example be cloned into any of a variety of cloning vectors, such as plasmid, phagemids, lambda phage derivatives and cosmids. Furthermore vectors such as expression vectors, replication vectors, probe generation vectors and sequencing vectors may be joined with the sequences disclosed herein.

Sequences that may be cloned to the nucleic acids according to the present invention comprise as well coding sequences as non-coding sequences and regulatory sequences including promoters, enhancers and terminators. The TKTL1 nucleic acid sequences disclosed herein might for example be present in combination with other coding sequences. These sequences may encode for a variety of proteins such as enzymes, receptors, antigens, immunogenic fragments or epitopes, binding proteins, etc.. The nucleic acid sequences may be joined directly or may be separated by a stretch of nucleic acids coding for a spacer or linker region. The nucleic acid sequences may also be separated by a stretch of nucleic acids that may be removed after transcription of the sequence. Non-coding sequences, that may be joined to the sequences disclosed herein may for example be promoter regions, enhancers, cis regulatory elements, 5' untranslated regions, terminators etc..

TKTL1 polynucleotides may be formulated such, that they are able to enter prokaryotic or eukaryotic cells such as mammalian cells and to be expressed in said cells. Such formulations may be useful for therapeutic purposes. The expression of nucleic acid sequences in target cells may be achieved by any method known to those skilled in the art. The nucleic acids may for example be joined to elements that are apt to enable their expression in a host cell. Such elements may comprise promoters or enhancers, such as CMV-, SV40-, RSV-, metallothionein I- or polyhedrin-promoters respectively CMV- or SV40-enhancers. Possible methods for the expression are for example incorporation of the polynucleotide into a viral vector including adenovirus, adeno-associated virus, retrovirus, vaccinia virus or pox virus. Viral vectors for the purpose of expression of nucleic acids in mammalian host cells may comprise pcDNA3, pMSX, pKCR, pEFBOS, cDM8, pCEV4 etc.. These techniques are known to those skilled in the art.

Fragments of the TKTL1 sequence used herein may comprise oligonucleotides such as nucleic acid probes for hybridisation purposes, primers for amplification reactions or antisense constructs for use in antisense techniques. Nucleic acid probes according to the present invention may be any nucleic acid probe that has a sequence at least 80% identical to a part of at least 15 consecutive nucleotides of the TKTL1 gene nucleic acid sequence or is complementary or reverse complementary to such a sequence but does not hybridise to an other transketolase or transketolase like sequence. The nucleic acid probes according to the present invention are furthermore characterized, in that they hybridise under stringent conditions to nucleic acids of the sequence disclosed herein. Primers may be any nucleotides that are suitable for carrying out a specific amplification reaction. Thus the primers used according to the present invention may be nucleic acid oligomers of at least 15 consecutive nucleotides with a sequence identity of at least 80% compared to the TKTL1 gene sequence or may be complementary or reverse complementary to such a sequence.

The primers according to the present invention specifically hybridise to the sequence disclosed herein or a part thereof under conditions suitably applied in the course of a nucleic acid amplification reaction but do not hybridise to an other transketolase or transketolase like sequence. Antisense oligonucleotides as used herein may be nucleic acid molecules reverse complementary to the transcripts of the disclosed coding sequence, that are able to bind to the transcripts by base pairing and such inhibit or reduce expression of said coding sequence.

The nucleic acids used according to the present invention may also be chemically pre-treated nucleic aids. Such chemically pre-treated nucleic acids may comprise any nucleic acid as disclosed herein, which has been treated with a chemical agent suitable to result in modifications in the nucleic acid molecules. Said modifications may for example comprise specific modifications of particular bases within the nucleic acid. Such chemical treatments may comprise treatment with e.g. sodium bisulphite, hydrazine or potassium permanganate. The sequences of special interest in experiments using chemical pre-treatment of nucleic acids may for example comprise coding or non-coding regions of the sequences. Examples of non-coding regions that may be treated by chemicals are promoter regions or CpG islands in 5' UTRs.

TKTL1 polypeptides as used according to the present invention may comprise amino acid chains of any length, including full length proteins, wherein the amino acid residues are linked by covalent peptide bonds. Thus, a polypeptide comprising a portion of one of the above TKTL1 proteins, e.g. a protein comprising the amino acid sequence of TKTL1 protein, may consist entirely of the protein, or the protein may be present within a larger polypeptide that contains additional sequences. The additional sequences may be derived from the native protein or may be heterologous, and such sequences may (but need not) be immunoreactive and/or antigenic. As detailed below, such polypeptides may be isolated from sample tissue or prepared by synthetic or recombinant means.

As used herein, a polypeptide exhibiting a biological activity of the TKTL1 polypeptide is understood to be a polypeptide having at least one of the activities, such as enzymatic activities (transketolase activity), inter protein interaction activities, responsiveness of the enzymatic activity to thiamine presence, or antigenic or immunogenic properties e.g. capability of binding an antibody directed against said polypeptide (i.e. comprising an immunogenic portion) of said TKTL1 polypeptide.

Immunogenic portion as used herein is a portion of a protein that is recognized by a B-cell and/or T-cell surface antigen receptor. The immunogenic portions comprise at least 5 amino acid residues, more preferably at least 10 amino acid residues and most preferably at least 15 amino acid residues of the protein disclosed herein. In a preferred embodiment of the present invention, particular domains of the protein, such as, for example, transmembrane domains or N-terminal leader sequences have been deleted.

The immunogenic portions according to the present invention react with antisera or specific antibodies in the same or nearly same intensity as the native full length proteins. The immunogenic portions are generally identified using the techniques well known in the art. Possible techniques are for example screening of the polypeptides for the ability to react with antigen-specific antibodies, antisera and/or T-cell lines or clones.

The TKTL1 polypeptides used according to the present invention comprise also variants of the native proteins.

These variants may differ from the native protein in one or more alterations such as substitutions, deletions, additions and/or insertions. The immunoreactivity and or biological activity of the variants according to the present invention is not substantially diminished compared to the native proteins. In a preferred embodiment of the invention the immunoreactivity and or activity is diminished less than 50%, in a more preferred embodiment the immunoreactivity and or activity is diminished less than 20 % compared to the native polypeptides. In another preferred embodiment of the present invention the variants of the polypeptides may be varied, such that the activity of the native protein is increased, decreased or lost. These variants may for example be employed in the therapy of disorders associated with the overexpression of TKTL1 gene. In a preferred embodiment, variants may be deficient in one or more portions, such as for example N-terminal leader sequences, transmembrane domains or small N- and/or C-terminal sequences. The variants exhibit preferably 70%, more preferably at least 90%, and most preferably at least 95% identity to the polypeptides disclosed according to the present invention.

The variants used according to the present invention comprise preferably conservative substitutions, so that the amino acids changed are substituted for amino acids with similar properties. The properties concerned may include polarity, charge, solubility, hydrophobicity, hydrophilicity and/or amphipathic nature of the amino acid residues.

The variants used according to the invention may also comprise additional terminal leader sequences, linkers or sequences, which enable synthesis, purification or stability of the polypeptides in an easier or more comfortable way.

Variants of TKTL1 may be produced by means of conventional molecular biological processes (see, e.g., Sambrook et al., 1989, Molecular Cloning, A Laboratory Manual, 2nd edition Cold Spring Harbor Laboratory Press, Cold Spring Harbor, NY). For example, it is possible to introduce different mutations into the nucleic acid molecules of the invention. As a result a TKTL1 polypeptide with possibly modified biological properties may be synthesized. One possibility is the production of deletion mutants in which nucleic acid molecules are produced by continuous deletions from the 5'-or 3'-terminal of the coding DNA sequence and that lead to the synthesis of polypeptides that are shortened accordingly. Another possibility is the introduction of single-point mutations at positions where a modification of the amino acid sequence influences, e.g., the enzyme activity or the regulation of the enzyme. By this method muteins can be produced, for example, that possess a modified Km-value or that are no longer subject to the regulation mechanisms that normally exist in the cell, e.g. with regard to allosteric regulation or covalent modification. Such muteins might, e.g., be valuable as therapeutically useful compounds, e.g. antagonists.

For the manipulation in prokaryotic cells by means of genetic engineering the nucleic acid molecules used for the methods of the invention or parts of these molecules can be introduced into plasmids allowing a mutagenesis or a modification of a sequence by recombination of DNA sequences. By means of conventional methods (cf. Sambrook et al., supra) bases may be exchanged and natural or synthetic sequences may be added. In order to link the DNA fragments with each other, adapters or linkers may be added to the fragments. Furthermore, manipulations may be performed that provide suitable cleavage sites or that remove superfluous DNA or cleavage sites. If insertions, deletions or substitutions are possible, in vitro mutagenesis, primer repair, restriction or ligation may be performed. As analysis method usually sequence analysis, restriction analysis and other biochemical or molecular biological methods may be used.

The polypeptides may comprise fusion or chimeric polypeptides containing sequences disclosed herein. Fusion proteins comprise TKTL1, a portion thereof or variants of the inventive polypeptide or portions thereof together with any second and further polypeptides, such as once more the inventive polypeptide, a portion thereof or variants of the inventive polypeptide or portions thereof and/or any heterologous polypeptides. Heterologous polypeptides may comprise enzymes, receptor molecules, antigens, antigenic or immunogenic epitopes or fragments thereof, antibodies or fragments thereof, signalling polypeptides or signal transducing polypeptides etc.. The immunogenic protein may, for example, be capable of eliciting a recall response. Examples of such proteins include tetanus, tuberculosis and hepatitis proteins (see, for example, Stoute et al. New Engl. J. Med., 336:86-91 (1997)). In one embodiment of the invention the fusion peptides may be constructed for enhanced detection or purification of the polypeptides. For the purpose of purification tags, such as e.g. his-tags, myc-tags etc. may be added to the polypeptides. For the purpose of detection antigenic portions, enzymes, chromogenic sequences etc. may be fused to the polypeptides. The fusion proteins of the present invention may (but need not) include a linker peptide between the first and second polypeptides.

A nucleic acid sequence encoding a fusion protein used in the present invention is constructed using known recombinant DNA techniques to assemble separate nucleic acid sequences encoding the first and second polypeptides into an appropriate expression vector. The 3' end of a nucleic acid sequence encoding the first polypeptide is ligated, with or without a peptide linker, to the 5' end of a nucleic acid sequence encoding the second polypeptide ensuring the appropriate reading frames of the sequences to permit mRNA translation of the two nucleic acid sequences into a single fusion protein that retains the biological activity of both the first and the second polypeptides.

A peptide linker sequence may be employed to separate the first and the second polypeptides by a distance sufficient to ensure, that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Pat. No. 4,935,233 and U.S. Pat. No. 4,751,180. The linker sequence may be from 1 to about 50 amino acids in length. Peptide sequences are not required when the first and second polypeptides have non-essential N-terminal amino acid regions that can be used to separate the functional domains and prevent steric interference.

The TKTL1 polypeptides for use in the method according to the present invention and nucleic acids encoding such polypeptides, may be isolated from tissue using any of a variety of methods well known in the art. Nucleic acid sequences corresponding to a gene (or a portion thereof) encoding one of the inventive polypeptides may be isolated from a cDNA library using a subtraction technique. Partial nucleic acid sequences thus obtained may be used to design oligonucleotide primers for the amplification of full-length nucleic acid sequences from a human genomic nucleic acid library or from a cDNA library in a polymerase chain reaction (PCR), using techniques well known in the art (see, for example, Mullis et al., Cold Spring Harbor Symp. Quant. Biol. 51:263, 1987; Erlich ed., PCR Technology, Stockton Press, NY, 1989). For this approach, sequence-specific primers may be designed based on the nucleotide sequences provided herein and may be purchased or synthesized.

The TKTL1 polypeptides used for the method disclosed herein may also be generated by synthetic means. In particular, synthetic polypeptides having fewer than about 100 amino acids, and generally fewer than about 50 amino acids, may be generated using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain (see, for example, Merrifield, J. Am. Chem. Soc. 85:2149-2146, 1963). Equipment for automated synthesis of polypeptides is commercially available from suppliers such as Perkin Elmer/Applied BioSystems Division (Foster City, Calif.), and may be operated according to the manufacturer's instructions.

The ligated nucleic acid sequences encoding the polypeptides used for the methods disclosed herein are operably linked to suitable transcriptional or translational regulatory elements known to the person skilled in the art. The regulatory elements responsible for expression of nucleic acid may be located e.g. 5' to the nucleic acid sequence encoding the first polypeptides, within the coding sequences or 3' to the nucleic acid sequences encoding the first or any further polypeptide. Stop codons required to end translation and transcription termination signals are present 3' to the nucleic acid sequence encoding the second polypeptide.

The polypeptides used for the methods according to the present invention may be isolated. This means that the molecules may be removed from their original environment. Naturally occurring proteins are isolated if they are separated from some or all of the materials, which coexist in the natural environment. Polynucleotides are isolated for example if they are cloned into vectors.

In certain preferred embodiments, described in more detail below, the polypeptides used in a method as disclosed herein may be prepared in an isolated, substantially pure form (i.e., the polypeptides are homogenous as determined by amino acid composition and primary sequence analysis). Preferably, the polypeptides are at least about 90% pure, more preferably at least about 95% pure and most preferably at least about 99% pure. The substantially pure polypeptides may for example be employed in pharmaceutical compositions.

Furthermore the present invention makes use of binding agents that specifically bind to a TKTL1 protein. These binding agents may comprise for example antibodies and antigen-binding fragments, bifunctional hybrid antibodies, peptidomimetics containing minimal antigen-binding epitopes etc.

An antibody or antigen-binding agent is said to react specifically, if it reacts at a detectable level with a protein used herein, and does not significantly react with other proteins. The antibodies according to the present invention may be monoclonal or polyclonal antibodies. As used herein, the term antibody or monoclonal antibody is meant to include intact molecules as well as antibody fragments (such as, for example, Fab and F(ab')2 fragments), which are capable of specifically binding to protein. Fab and F(ab')2 fragments lack the Fc fragment of intact antibody, clear more rapidly from the circulation, and may have less non-specific tissue binding than an intact antibody (Wahl et al., J. Nucl. Med. 24:316-325 (1983). Thus, these fragments are preferred, as well as the products of a Fab or other immunoglobulin expression library. Moreover, antibodies used in the present invention include chimeric, single chain, and humanized antibodies.

According to the present invention binding agents may be used isolated or in combination. By means of combination it is possible to achieve a higher degree of sensitivity. The term antibody, preferably, relates to antibodies, which consist essentially of pooled monoclonal antibodies with different epitopic specificities, as well as distinct monoclonal antibody preparations.

Monoclonal antibodies are made from an antigen containing fragments of the TKTL1 polypeptide using any of a variety of techniques known to those of ordinary skill in the art; see, e.g., Harlow and Lane, Antibodies: A Laboratory Manual, Cold Spring Harbor Laboratory, 1988. In one such technique, an immunogen comprising the antigenic polypeptide or a synthetic part thereof is initially injected into any of a wide variety of mammals (e.g., mice, rats, rabbits, sheep and goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for the antigenic polypeptide of interest may be prepared, for example, using the technique of Köhler and Milstein, Eur. J. Immunol. 6:511-519, 1976, and improvements thereto. Briefly, these methods involve the preparation of immortal cell lines capable of producing antibodies having the desired specificity (i.e., reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a non-ionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The TKTL1 polypeptides may be used in the purification process in, for example, an affinity chromatography step.

The TKTL1 specific antibodies used according to the present invention may comprise further binding sites for either therapeutic agents or other polypeptides or may be coupled to said therapeutic agents or polypeptides. Therapeutic agents may comprise drugs, toxins, radio nuclides and derivatives thereof. The agents may be coupled to the binding agents either directly or indirectly for example by a linker or carrier group. The linker group may for example function in order to enable the coupling reaction between binding agent and therapeutic or other agent or the linker may act as a spacer between the distinct parts of the fusion molecule. The linker may also be cleavable under certain circumstances, so as to release the bound agent under said conditions. The therapeutic agents may be covalently coupled to carrier groups directly or via a linker group. The agent may also be non-covalently coupled to the carrier. Carriers that can be used according to the present invention are for example albumins, polypeptides, polysaccharides or liposomes.

The TKTL1 specific antibodies used according to the present invention may be coupled to one or more agents. The multiple agents coupled to one antibody may be all of the same species or may be several different agents bound to one antibody.

The methods disclosed herein are applicable to all eukaryotic organisms prone to be affected by disorders associated with the dysfunction of a transketolase like-1 or a TKTL1 encoding gene. Individuals as used in the context of the present invention may for example comprise mammals, such as animals of agricultural interest (cows, sheep, horses, pigs, etc.), companion animals (cats, dogs, etc.), animals commonly employed for research use (rats, mice, hamsters, etc.) or human beings.

Diagnosis as used in the context of the present invention may comprise determining the level of TKTL1 gene products or determining the enzymatic activity of TKTL1 in a sample. Based upon the determined level of TKTL1 gene products or the determined enzymatic activity in the samples individuals can be subdivided into subgroups. Based upon these subgroups an assessment of prognosis may be done. According to the subgroups the therapy of the individuals affected by the various diseases may be tailored. For example the overexpression of TKTL1 gene and an enhanced activity of the pentose-phosphate cycle suggest a mechanism by which thiamine (vitamin B1) promotes nucleic acid ribose synthesis and an enhanced glucose metabolism. Therefore the thiamine intake has direct consequences for a disease with an overexpression of the transketolase like-1 gene. This provides also background information and helps to develop guidelines for alternative treatments with antithiamine transketolase inhibitors in the clinical setting. Analysis of RNA ribose indicates that glucose carbons contribute to over 90% of ribose synthesis in cultured cervix und pancreatic carcinoma cells and that ribose is synthesized primarily through the thiamine dependent transketolase pathway (> 70%). Antithiamine compounds significantly inhibit nucleic acid synthesis. Thiamine or bentotiamine treatment activates TKTL1 and thereby the sugar metabolism and reduces toxic or unwanted reactions (e.g. AGE formation, glyoxal.

Thus, based on the detection of overexpression or reduced expression or enhanced or reduced TKTL1 enzyme activity new treatment strategies may be tailored targeting specific biochemical reactions of pentose-phosphate cycle by hormones related to glucose metabolism, controlling thiamine intake, the cofactor of the nonoxidative transketolase pentose-phosphate cycle reaction, or treating patients with antithiamine analogues or thiamin or thiamin derivates (e.g. benfotiamine).

Thus, in one embodiment of the invention disorders characterized by reduced or enhanced TKTL1 expression or reduced or enhanced enzymatic activity, the TKTL1 gene may be treated in accordance to the level of expression of TKTL1 gene. Using the non-oxidative pentose-phosphate cycle reactions to inhibit glucose utilizing pathways selectively for nucleic acid production offers a new target site for treatment with a strong regulatory effect on the cell cycle and glucose metabolism.

In one embodiment the treatment of disorders associated with overexpression of TKTL1 gene may comprise restricted or enhanced administration of thiamine (benfotiamine or other thiamine derivatives) to the affected individuals. In another embodiment the treatment may comprise the administration of TKTL1 inhibitors, such as e.g. antithiamine compounds.

Examples of TKTL1 inhibitors according to the invention are oxythiamine, benfooxytiamine, hydroxypyruvate, pyruvate, p-hydroxyphenylpyruvate, pyrithiamin, amprolium, 2-methylthiamin, benfotiamine, benfooxytiamine, 2-methoxy-*p*-benzochinon (2-MBQ) und 2,6-dimethoxy-p-benzochinon (2,6-DMBQ), genistein, and flavonols as e.g. quercetin, catechins, nitrilosides, anthocyanins and derivatives thereof.

Derivatives of the above listed activators or inhibitors can be generated by substituting or adding one or more of the following groups:
linear and branched (C₁-C₁₂) aliphatic alkyl groups, substituted with at least one group chosen from OH, NH₂, SH, CN, CF₃, halogen, CONHR⁵, COOR⁵, OR⁵, SR⁵, SiOR⁵, NHR⁵, aliphatic (C₃-C₆) rings, and aromatic (C₃-C₆) rings, wherein R⁵ is chosen from linear and branched (C₁-C₄) alkyl groups,
aryl groups,
natural polymers, synthetic polymers, and copolymers, said polymers and copolymers carrying at least two groups chosen from: hydroxyl, carboxylate, primary amine, secondary amine, tertiary amine, thiol, and aldehyde;
a hydrogen atom, a halogen atom, CF₃, OH, OCF₃, COOH, R⁷, OR₇, and OCOR⁷, wherein R⁷ is chosen from linear and branched (C₁₋C₄) aliphatic alkyl groups;
a monohalogenated and polyhalogenated linear and branched (C₁-C₄) alkyl groups, and from aryl groups, wherein the aryl groups are optionally substituted with at least one group chosen from OH, NH₂, SH, CN, CF₃, halogen, COOH, CONHR⁸, COOR⁸, OR⁸, SR⁸, and NHR⁸, wherein R⁸ is chosen from linear and branched (C₁-C₁₂) alkyl radicals; and
from linear and branched (C₁-C₄) alkyl groups, and a CF₃ group.

Monitoring may comprise detecting the level of TKTL1 gene products or TKTL1 enzymatic activity in samples taken at different points in time and determining the changes in said level. According to said changes the course of the disease can be followed. The course of the disease may be used to select therapy strategies for the particular individual.

Another aspect of diagnosis and monitoring of the disease course according to the present invention may comprise the detection of minimal residual disease. This may comprise for example the detection of a TKTL1 gene products level or TKTL1 enzymatic activity in one or more body samples following initial therapy of an individual once or at several time points. According to the level of TKTL1 gene products detected in the samples one may select a suitable therapy for the particular individual.

A sample according to the method of the present invention may comprise any sample comprising cells or cell debris. Samples may comprise samples of clinical relevance, such as e.g. secretions, such as gastric juice, bile or pancreatic juice, smears, body fluids, such as serum, blood, plasma urine, semen, stool, biopsies or cell- and tissue-samples. Biopsies as used in the context of the present invention may comprise e.g. resection samples, tissue samples prepared by endoscopic means or needle biopsies of organs. Furthermore any sample potentially containing the marker molecules to be detected may be a sample according to the present invention.

Such samples may comprise for example intact cells, lysed cells or any liquids containing proteins, peptides or nucleic acids. Even solids, to which cells, cell fragments or marker molecules, such as TKTL1 nucleic acids or TKTL1 proteins, may adhere may be samples according to the present invention. Such solids may comprise for example membranes, glass slides, beads etc..

Preparation of a sample may comprise e.g. obtaining a sample of a tissue, a body fluid, of cells, of cell debris from a patient. According to the present invention preparation of the sample may also comprise several steps of further preparations of the sample, such as preparation of dissections, preparation of lysed cells, preparation of tissue arrays, isolation of polypeptides or nucleic acids, preparation of solid phase fixed peptides or nucleic acids or preparation of beads, membranes or slides to which the molecules to be determined are coupled covalently or non-covalently.

The method for detection of the level of the TKTL1 gene product according to the present invention is any method, which is suited to detect very small amounts of specific biologically active molecules in biological samples. The detection reaction according to the present invention is a detection either on the level of nucleic acids or on the level of polypeptides.

The detection may be carried out in solution or using reagents fixed to a solid phase. The detection of one or more molecular markers, such as polypeptides or nucleic acids, may be performed in a single reaction mixture or in two or separate reaction mixtures. Alternatively, the detection reactions for several marker molecules may, for example, be performed simultaneously in multi-well reaction vessels. The markers characteristic for the TKTL1 gene products may be detected using reagents that specifically recognise these molecules. The detection reaction for the marker molecules may comprise one or more reactions with detecting agents either recognizing the initial marker molecules or recognizing the prior molecules used to recognize other molecules.

The detection reaction further may comprise a reporter reaction indicating the presence or absence and/or the level of the TKTL1 gene markers. The reporter reaction may be for example a reaction producing a coloured compound, a bioluminescence reaction, a fluorescence reaction, generally a radiation emitting reaction etc.. In a preferred embodiment, different marker molecules may be recognized by agents that produce different reporter signals, so that the signals referring to marker molecules could be distinguished.

Applicable formats for the detection reaction according to the present invention may be, blotting techniques, such as Western-Blot, Southern-blot, Northern-blot and ELISA. The blotting techniques are known to those of ordinary skill in the art and may be performed for example as electro-blots, semidry-blots, vacuum-blots or dot-blots. Amplification reactions may also be applicable for the detection of e.g. nucleic acid molecules. Furthermore immunological methods for detection of molecules may be applied, such as for example immunoprecipitation or immunological assays, such as ELISA, RIA, lateral flow assays, immuno-cytochemical methods etc..

In one preferred embodiment of the invention the detection of the level of TKTL1 gene products is carried out by detection of the level of nucleic acids coding for the TKTL1 gene products or fragments thereof present in the sample. The means for detection of nucleic acid molecules are known to those skilled in the art. The procedure for the detection of nucleic acids can, for example, be carried out by a binding reaction of the molecule to be detected to complementary nucleic acid probes, proteins with binding specificity for the nucleic acids or any other entities specifically recognizing and binding to said nucleic acids. This method can be performed as well in vitro as directly in situ for example in the course of a detecting staining reaction. Another way of detecting the TKTL1 gene products in a sample on the level of nucleic acids performed in the method according to the present invention is an amplification reaction of nucleic acids, which can be carried out in a quantitative manner such as for example the polymerase chain reaction. In a preferred embodiment of the present invention real time RT PCR may be used to quantify the level of TKTL1 RNA in samples.

In another preferred embodiment of the invention the detection of the level of TKTL1 gene products is carried out by determining the level of expression of a protein. The determination of the TKTL1 gene products on the protein level can for example be carried out in a reaction comprising an antibody specific for the detection of the TKTL1 protein. The antibodies can be used in many different detection techniques for example in Western-blot, ELISA or immunoprecipitation. Generally antibody based detection can be carried out as well in vitro as directly in situ for example in the course of an immuno-histochemical staining reaction. Any other method for determining the amount of particular polypeptides in biological samples can be used according to the present invention.

In one preferred embodiment of the invention the level of TKTL1 gene products is significantly elevated compared to a control test sample. In this case the TKTL1 gene is overexpressed in the sample.

One example for the diagnosis of disorders associated with the (over)expression of the TKTL1 gene may comprise the detection of auto-antibodies directed against polypeptides encoded by the TKTL1 gene. The polypeptides used for the methods according to the present invention may be used to detect the presence or absence of such antibodies in body fluids by methods known to those of skill in the art.

In one preferred embodiment the detection of tissues expressing TKTL1 gene products is carried out in form of molecular imaging procedures. The respective procedures are known to those of ordinary skill in the art. Imaging methods for use in the context of the present invention may for example comprise MRI, SPECT, PET and other methods suitable for in vivo imaging.

In one embodiment the method may be based on the enzymatic conversion of inert or labelled compounds to molecules detectable in the course of molecular imaging methods by the TKTL1 molecules. In another embodiment the molecular imaging method may be based on the use of compounds carrying a suitable label for in vivo molecular imaging, such as radio isotopes, metal ions etc., specifically binding to TKTL1 molecules in vivo.

In a preferred embodiment of the invention these compounds are non-toxic compounds and may be eliminated from the circulation of organisms, such as humans, in a time span, that allows for performing the detection of label accumulated in tissue overexpressing TKTL1 gene. In another preferred embodiment of the invention compounds are used for molecular imaging, for which clearance from the circulation is not relevant for performing the molecular imaging reaction. This may be for example due to low background produced by the circulating molecules etc. The compounds for use in molecular imaging methods are administered in pharmaceutical acceptable form in compositions that may additionally comprise any other suitable substances, such as e.g. other diagnostically useful substances, therapeutically useful substances, carrier substances or the like.

The reagent for the detection of the TKTL1 gene may include any agent capable of binding to the TKTL1 molecule. Such reagents may include proteins, polypeptides, nucleic acids, peptide nucleic acids, glycoproteins, proteoglycans, polysaccharides or lipids.

The TKTL1 sample for carrying out a positive control may comprise for example TKTL1 nucleic acids or polypeptides or fragments thereof in applicable form, such as solution or salt, peptides in applicable form, tissue section samples or positive cells.

In a preferred embodiment of the invention the detection of the TKTL1 gene product is carried out on the level of polypeptides. In this embodiment the binding agent may be for example an antibody specific for the TKTL1 or a fragment thereof.

In another embodiment, the detection of the TKTL1 gene products is carried out on the nucleic acid level. In this embodiment of the invention the reagent for the detection may be for example a nucleic acid probe or a primer reverse-complementary to said TKTL1 nucleic acid.

In addition to TKTL1 protein variants with enhanced or reduced levels of enzymatic activity, TKTL1 proteins with altered localization or aggregation and/or dimerization within the cell can be detected in patients. Using monoclonal antibodies specifically detecting TKTL1 protein (Linaris Biologische Produkte, Wertheim) TKTL1 protein within the nucleus can be detected in cells isolated from body fluids. Using immunocytochemistry the localization of TKTL1 within the nucleus and the cytoplasm of cells from healthy individuals and patients can be determined. In a subset of Alzheimer patients an enhanced localization of TKTL1 is detectable within the nucleus. A different aggregation of TKTL1 in Alzheimer patients is also present. Detection of this aggregation is possible using 2D-gel electrophoresis (Fig. 15). In addition an aggregation with other proteins e.g. GAPDH can be detected by an ELISA using antibodies for TKTL1 and GAPDH.

In a further aspect the present invention relates to the use of one or more of the compounds useful for the methods according to the present invention such as a nucleic acid molecule, a recombinant vector, a polypeptide, an antisense RNA sequence, a ribozyme or an antibody for the preparation of a pharmaceutical composition for the prevention or treatment of a disease associated with an enhanced or decreased level or activity, an abnormal cellular localization of the mutated TKTL1 protein, aggregation status and/or dimerization status compared to a control contraception.

The polypeptides, polynucleotides and binding agents useful in the method according to the present invention may be incorporated into pharmaceutical or immunogenic compositions. The pharmaceutical compositions comprise said compounds and a physiologically acceptable carrier.

A pharmaceutical composition or vaccine may for example contain DNA that codes for a functional TKTL1. The DNA may be administered in a way that allows the polypeptides to be generated in situ. Suitable expression systems are known to those skilled in the art. The expression of the polypeptides may for example be persistent or transient. In pharmaceutical compositions and/or vaccines, providing for in-situ expression of polypeptides, the nucleic acids may be present within any suitable delivery system known to those of ordinary skill in the art, including nucleic acid expression systems, bacteria and viral expression systems. Appropriate nucleic acid expression systems comprise the necessary regulatory nucleic acid sequences for expression in the patient, such as suitable promoters, terminators etc.. Bacterial delivery systems may for example employ the administration of a bacterium that expresses an epitope of a cell antigen on its cell surface. In a preferred embodiment, the nucleic acid may be introduced using a viral expression system such as e.g., vaccinia virus, retrovirus, or adenovirus, which may involve the use of a non-pathogenic, replication competent virus. Suitable systems are disclosed, for example, in Fisher-Hoch et al., PNAS 86:317-321, 1989; Flexner et al., Ann. N.Y. Acad Sci. 569:86-103, 1989; Flexner et al., Vaccine 8:17-21, 1990; U.S. Pat. Nos. 4,603,112, 4,769,330, and 5,017,487; WO 89/01973; U.S. Pat. No. 4,777,127; GB 2,200,651; EP 0,345,242; WO 91/02805; Berkner, Biotechniques 6:616-627, 1988; Rosenfeld et al., Science 252:431-434, 1991; Kolls et al., PNAS 91:215-219, 1994; Kass-Eisler et al., PNAS 90:11498-11502, 1993; Guzman et al., Circulation 88:2838-2848, 1993; and Guzman et al., Cir. Res. 73:1202-1207, 1993.

In another embodiment transgenic mammalian cells may be used for delivery and/or expression of the nucleic acids. The methods for producing nucleic acid constructs suitable for in-situ expression of polypeptides are known to those of skill in the art.

In another embodiment of the invention the nucleic acids may be for example anti-sense constructs. The nucleic acid may also be administered as a naked nucleic acid. In this case appropriate physical delivery systems, which enhance the uptake of nucleic acids may be employed, such as coating the nucleic acid onto biodegradable beads, which are efficiently transported into the cells. Administration of naked nucleic acids may for example be useful for the purpose of transient expression within a host or host cell.

Alternatively, the pharmaceutical compositions may comprise one or more polypeptides. The polypeptides incorporated into pharmaceutical compositions may be the TKTL1 polypeptides in combination with one or more other known polypeptides such as for example enzymes, antibodies, regulatory factors, such as cyclins, cyclin-dependent kinases or CKIs, or toxins.

The pharmaceutical compositions may be administered by any suitable way known to those of skill in the art. The administration may for example comprise injection, such as e.g., intracutaneous, intramuscular, intravenous or subcutaneous injection, intranasal administration for example by aspiration or oral administration. A suitable dosage to ensure the pharmaceutical benefit of the treatment should be chosen according the parameters, such as age, sex, body weight etc. of the patient, known to those of skill in the art.

The type of carrier to be employed in the pharmaceutical compositions will vary depending on the mode of administration. For parenteral administration, such as subcutaneous injection, the carrier preferably comprises water, saline, alcohol, a lipid, a wax and/or a buffer. For oral administration, any of the above carriers or a solid carrier, such as mannitol, lactose, starch, magnesium stearate, sodium saccharine, talcum, cellulose, glucose, sucrose, and/or magnesium carbonate, may be employed. Biodegradable microspheres (e.g., polylactic glycolide) may also be employed as carriers for the pharmaceutical compositions of this invention. Suitable biodegradable microspheres are disclosed, for example, in U.S. Pat. Nos. 4,897,268 and 5,075,109.

The compounds of the present invention may furthermore be incorporated into immunogenic compositions. The constituents of an immunogenic composition may comprise vaccines, antigens, antigenic fragments or nucleic acids coding for antigens or antigenic fragments to be expressed in situ. These compounds may be present as polypeptides, or as nucleic acids, that allow the polypeptides to be expressed in situ. Immunogenic compositions comprise said compounds and additionally an immunostimulant or immunogenic adjuvant.

Polypeptides of the present invention or fragments thereof, that comprise an immunogenic portion of a TKTL1 protein, may be used in immunogenic compositions, wherein the polypeptide e.g. stimulates the patient's own immune response. A patient may be afflicted with disease, or may be free of detectable disease. Accordingly, the compounds disclosed herein may be used to treat or to prevent the development of a disease.

Immunogenic compositions such as vaccines may comprise one or more polypeptides and a non-specific immune-response enhancer, wherein the non-specific immune response enhancer is capable of eliciting or enhancing an immune response to an exogenous antigen. Examples of non-specific immune response enhancers include adjuvants, biodegradable microspheres (e.g., polylactic galactide) and for example liposomes into which the polypeptide may be incorporated. Pharmaceutical compositions and vaccines may also contain other epitopes of tumor antigens, either incorporated into a fusion protein as described above (i.e., a single polypeptide that contains multiple epitopes) or present within a separate polypeptide.

Any suitable immune-response enhancer may be employed in the vaccines of this invention. For example, an adjuvant may be included. Most adjuvants contain a substance designed to protect the antigen from rapid catabolism, such as aluminium hydroxide or mineral oil, and a non-specific stimulator of immune response, such as lipid A, Bordetella pertussis or Mycobacterium tuberculosis. Such adjuvants are commercially available as, for example, Freund's Incomplete Adjuvant and Complete Adjuvant (Difco Laboratories, Detroit, Mich.) and Merck Adjuvant 65 (Merck and Company, Inc., Rahway, N.J.).

For therapeutic purposes, polypeptides, polynucleotides or binding agents may be administered in a variety of ways. Possible ways may for example comprise intracutaneous, intramuscular, intravenous or subcutaneous injection, intranasal administration for example by aspiration or oral administration.

Therapy can be carried out using TKTL1 polypeptides and/or polynucleotides. The therapy may be for example immunotherapy or somatic gene therapy. The TKTL1 polypeptides and/or polynucleotides may according to the present invention be used for vaccination. Vaccination according to the present invention may comprise administering an immunogenic compound to an individual for the purpose of stimulating an immune response directed against said immunogenic compound and thus immunizing said individual against said immunogenic compound. Stimulating an immune response may comprise inducing the production of antibodies against said compound as well as stimulating cytotoxic T-cells. For the purpose of vaccination the polypeptides, nucleic acids and binding agents according to the present invention may be administered in a physiological acceptable form. The composition to be administered to individuals may comprise one or more antigenic components, physiologically acceptable carrier substances or buffer solutions, immunostimulants and/or adjuvants. Adjuvants may comprise for example Freund's incomplete adjuvant or Freund's complete adjuvant or other adjuvants known to those of skill in the art.

The composition may be administered in any applicable way such as e.g. intravenous, subcutaneous, intramuscular etc.. The dosage of the composition depends on the particular case and purpose of the vaccination. It has to be adapted to parameters by the individual treated such as age, weight, sex etc.. Furthermore the type of the immune response to be elicited has to be taken into account. In general it may be preferable if an individual receives 100 µg - 1 g of a polypeptide according to the present invention or 10⁶ - 10¹² MOI of a recombinant nucleic acid, containing a nucleic acid according to the present invention in a form that may be expressed in situ.

Vaccination of individuals may be favourable e.g. in the case of altered, non wild-type sequences or structure of marker molecules associated with cell proliferative disorders.

Further methods for treatment of disorders associated with overexpression of TKTL1 gene may comprise any method suitable for the reduction of the activity of TKTL1 polypeptide in an individual or in cells of an individual. These methods may comprise a reduction of the activity of TKTL1 polypeptide by means of reduction of gene expression or by means of reduction of enzymatic activity. Examples may comprise the administration of antisense constructs, of ribozymes, of enzyme inhibitors, the administration of antagonists of cofactors of TKTL1 polypeptides, such as e.g. antithiamine compounds or the reduced administration of essential cofactors for the enzymatic activity (e.g. thiamine).

The methods for administration of ribozymes or antisense constructs are known to those of skill in the art. The administration may take place as administration of naked nucleic acids or as administration of nucleic acids that are suited for expression of the relevant active products in situ.

In one preferred embodiment the therapy of disorders associated with the overexpression of TKTL1 gene comprises administration of antithiamine compounds, or the reduction of thiamine uptake for individuals showing disorders characterized by overexpression of TKTL1 gene.

The present invention also relates to the use of compound for the treatment of a disease associated with an enhanced or decreased level or activity, an abnormal cellular localization of the mutated TKTL1 protein, aggregation status and/or dimerization status compared to a control, wherein said compound is a compound identified by a method comprising the following steps:
(a) contacting a TKTL1 polypeptide as defined in the preceding claims or a cell expressing said polypeptide in the presence of components capable of providing a detectable signal in response to a biological activity, preferably transketolase activity; and
(b) detecting presence or absence of a signal or increase of the signal generated from said biological activity, wherein the absence, decrease or increase of the signal is indicative for a putative drug.

The drug candidate may be a single compound or a plurality of compounds. The term "plurality of compounds" in a method of the invention is to be understood as a plurality of substances which may or may not be identical. Said compound or plurality of compounds may be chemically synthesized or microbiologically produced and/or comprised in, for example, samples, e.g., cell extracts from, e.g., plants, animals or microorganisms. Furthermore, said compound(s) may be known in the art but hitherto not known to be capable of suppressing or activating TKTL1 polypeptides. The reaction mixture may be a cell free extract or may comprise a cell or tissue culture. Suitable set ups are known to the person skilled in the art and are, for example, generally described in Alberts et al., Molecular Biology of the Cell, third edition (1994) and in the appended examples. The plurality of compounds may be, e.g., added to the reaction mixture, culture medium, injected into a cell or otherwise applied to the transgenic animal. The cell or tissue that may be employed in the method of the invention preferably is a host cell, mammalian cell or non-human transgenic animal of the invention described in the embodiments hereinbefore.

If a sample containing a compound or a plurality of compounds is identified in the method of the invention, then it is either possible to isolate the compound from the original sample identified as containing the compound capable of suppressing or activating TKTL1, or one can further subdivide the original sample, for example, if it consists of a plurality of different compounds, so as to reduce the number of different substances per sample and repeat the method with the subdivisions of the original sample. Depending on the complexity of the samples, the steps described above can be performed several times, preferably until the sample identified according to the present method only comprises a limited number of or only one substance(s). Preferably said sample comprises substances of similar chemical and/or physical properties, and most preferably said substances are identical.

The compounds which can be tested and identified may be peptides, proteins, nucleic acids, antibodies, small organic compounds, hormones, peptidomimetics, PNAs or the like. These compounds may also serve as lead compounds for the development of analog compounds. The analogs should have a stabilized electronic configuration and molecular conformation that allows key functional groups to be presented to the TKTL1 in substantially the same way as the lead compound. In particular, the analog compounds have spatial electronic properties which are comparable to the binding region, but can be smaller molecules than the lead compound, frequently having a molecular weight below about 2 kD and preferably below about 1 kD. Identification of analog compounds can be performed through use of techniques such as self-consistent field (SCF) analysis, configuration interaction (CI) analysis, and normal mode dynamics analysis. Computer programs for implementing these techniques are available; e.g., Rein, Computer-Assisted Modeling of Receptor-Ligand Interactions (Alan Liss, New York, 1989).

Methods for the preparation of chemical derivatives and analogues are well known to those skilled in the art and are described, for example, in Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art; see also supra. Furthermore, peptidomimetics and/or computer aided design of appropriate derivatives and analogues can be used, for example, according to the methods described above.

Once the described compound has been identified and obtained, it is preferably provided in a therapeutically acceptable form.

Furthermore, the TKTL1 proteins play important roles in nutrient signaling in eukaryotic cells. Using a high througput screen, novel small molecules, small-molecule inhibitors of TKTL1 and small-molecule enhancers of TKTL1, that suppress and augment, respectively, TKTL1 effect on sugar metabolism can be identified.

Inhibitors of TKTL1 are, for example, oxythiamine, benfooxytiamine, hydroxypyruvate, pyruvate, p-hydroxyphenylpyruvate, pyrithiamin, amprolium, 2-methylthiamin, benfotiamine, benfooxytiamine, 2-methoxy-*p-*benzochinon (2-MBQ) und 2,6-dimethoxy-*p*-benzochinon (2,6-DMBQ), genistein, and flavonols as e.g. quercetin, catechins, nitrilosides and anthocyanins.

### TKTL1 and obesity

Reducing the TKTL1 activity can cause growth retardation and preferential reduction of adipose tissue. Obesity can be treated by inhibition of TKTL1 in adipose tissue.

The use of TKTL1 inhibitors identified by the methods according to the invention can be applied in tumour therapy and in restenosis in heart valves to prevent proliferation of endothelial cells.

### TKTL1 and autoimmundisease

Diseases like sytemic lupus erythematosus (SLE), rheumatoid arthritis, multiple sclerosis (MS), fibromyalgia, crohn's disease, irritable bowel syndrome (IBS) have steadily risen for the past 80 years. Targeting TKTL1 lead to a significant decrease in levels of auto-antibodies in patients with autoimmundiseases.

Unwanted apoptosis in cells like neurons can be reduced or blocked by treatment of TKTL1 with compounds identified by the methods according to the invention.

The following examples illustrate the invention.

### Example 1: Determining the level of TKTL1 mRNA levels in tissues

Dissections of biopsies can be semi-quantitatively analysed for the mRNA level of TKTL1 gene in an in-situ staining reaction. The staining reaction is performed as follows:

The tissue dissections are incubated in ascending ethanol concentrations up to 100% ethanol. After evaporation of the alcohol the dissections are boiled in 10 mM citrate buffer (pH 6,0) for pre-treatment of the tissue. The hybridisation mixture is prepared by mixing 50 µl of ready to use hybridisation buffer (DAKO A/S, Glostrup, Danmark) with about 5-10 pmol of the probes. The probes are fluorescein-labelled oligonucleotides of the following sequence: TCTCATCACAAGCAGCACAGGAC

### Example 2: Assays for detection of compounds for enhancing or reducing the TKTL1 enzyme activity

Since the TKTL1 protein isoforms represent moonlighting proteins, different assays for identifying active small compounds have to be applied. Assays for detection of compounds for enhancing or reducing the TKTL1 enzyme activity can be performed by the recombinant protein isoforms or by native protein isoforms isolated from human cells.

### (A) expression of a recombinant TKTL1 protein isoform in E.coli

The TKTL1 open reading frame (MADAE...CMLLN) of cDNA sequence (acc. no. BC025382) was cloned into the pDEST17 vector (Invitrogen). Bacterial expression was performed in the E. coli strain BL21-AI (Invitrogen), and expression was induced with 0.2% arabinose at 21°C for 4 h. Crude cell lysate was prepared in lysis buffer (20mM Tris[pH7.5], 5mM imidazole, 5 mM □-mercaptoethanol, 500 mM NaCl, and 1% Triton X-100) by freezing (dry ice, 10 min) and thawing (37°C, 5 min) 3 times. Soluble protein fractions were obtained by centrifugation of the cell lysate at 12.000 xg for 30 min at 4°C. His₆-TKTL1 protein was purified with Ni-NTA resins (Qiagen) according to the manufacturer's instructions with elution buffer containing 200 mM imidazole. Imidazole and salt were subsequently removed by dialysis against 0.1 M Tris (pH 7.5). The purified enzyme was stored at -20°C in 40% glycerol and 0.1% dithiothreitol (DTT).

### (B) Affinity-purification of native TKTL1 protein and protein complexes harboring TKTL1 from human cell lines

10 mg of MAb JFC12T10 was coupled to 2 ml carbo-link according to the manufacturer's instructions (carbo-link; Pierce). Cells were grown in serum free media (ISF-1, InVivo BioTech Services GmbH). After centrifugation, the pellet of 2.2x10⁹ cells was resolved in 50 ml PBS containing protease inhibitors cocktail (Roche). A cell lysis was performed using a french press, followed by a centrifugation at 50.000 xg. The supernatant was filtered (0.2 µm) and binding of supernatant to affinity material was performed over night at 4°C (batch modus). After transfer to a column, a wash procedure was performed with 150 mM PBS buffer pH 7.4. For elution of column attached proteins 100mM Glycin-HCl pH 2.0 was used. Two proteins peaks, detected using a UV 280 nm-based detection system, have been collected and neutralized with Tris pH 7.4.

Enymatic tests can be performed with the recombinant or the native, affinity purified TKTL1 protein.

### (C) Determining the two-substrate transketolase and one-substrate reaction.

The transketolase (two-substrate) activity of TKTL1 was measured by a coupled enzyme assay at 25°C. Reactions were started by addition of recombinant and native TKTL1 protein and determined spectrophotometrically by the rate of reduction of NAD⁺ in the following reaction sequence: xylulose-5-phosphate (X5P) and ribose-5-phosphate (R5P) > (TKTL1 activity) > glyceraldehyde-3-phosphate, sedoheptulose-7-phosphate > (glyceraldehyde-3-phosphate dehydrogenase activity [GAPDH]) > NAD⁺ -> NADH + H⁺, 1,3-phosphoglycerate.

Transketolase two-substrate activity was determined in the following reaction (final concentrations): 4 mM X5P, 4 mM R5P, 500 µM NAD⁺, 2 mM MgCl₂, 200 µM thiamine PP, 5 µg recombinant TKTL1 protein, or 4 µg native TKTL1 protein, 3 U GAPDH, 0.15 mol/l Tris buffer pH 7.4 in a reaction volume of 1 ml. Transketolase one-substrate activity was determined by omitting R5P, using X5P solely as substrate. GAPDH was obtained from Sigma.

Example C-2: Transketolase activity can be measured by using a conventional enzyme-linked method under conditions in which coupling enzymes are not limiting. Reactions are initiated by the addition of transketolase protein to an otherwise complete reaction mix of 100 mmol/L Tris-HC1 (pH 7,5), 10 mmol/L ribose 5-phosphate, 2 mmol/L xylulose 5-phophate, 1,2 mmol/L MgCl₂, 0,1 mmol/L NADH, 2000 U/L glycerol-3-phosphate dehydrogenase and triose phophate isomerase. Reactions are conducted at 37°C. The oxidation of NADH, which is directly proportional to transketolase activity, is followed by monitoring the decrease in absorbance at 340 nm.

Example C-3: Substrates (in variable concentrations) can be tested as possible donors if erythrose-4-phosphate (1 mM) as acceptor is used. In such a reaction fructose-6-phosphate will be build. With the enzymes glucose-6-phosphate-dehydrogenase and 6-phosphoglucose-isomerase, fructose-6-phosphate will be oxidized to 6-phosphogluconolactone, leading to the generation of NADPH.

Example C-4: Formaldehyde (variable concentrations) as acceptor can be used leading to dihydroxyacetone. The following reaction of glycerin-dehydrogenase builds glycerin, concomittant with an oxidation of NADH.

### Example C-5: Determining the one-substrate transketolase reaction

Transketolase activity is measured by using a conventional enzyme-linked method under conditions in which coupling enzymes are not limiting. Reactions are initiated by the addition of transketolase protein to an otherwise complete reaction mix of 100 mmol/L Tris-HC1 (pH 7,5), 5 mmol/L xylulose 5 phophate, 1,2 mmol/L MgCl₂, 3 mmol/L phosphate, 0,1 mmol/L NADH, 2000 U/L glycerol-3-phosphate dehydrogenase and triose phophate isomerase. Reactions are conducted at 37°C. The oxidation of NADH, which is directly proportional to transketolase activity, is followed by monitoring the decrease in absorbance at 340.

### Example C-6: Determining the one-substrate transketolase reaction

Transketolase activity is measured by using a conventional enzyme-linked method under conditions in which coupling enzymes are not limiting. Reactions are initiated by the addition of transketolase protein to an otherwise complete reaction mix of 100 mmol/L Tris-HCl (pH 7,5), 5 mmol/L xylulose 5 phophate, 3 mmol/L phosphate, 1,2 mmol/L MgCl₂, 0,1 mmol/L NAD, 2000 U/L glyceraldehyde-3-phosphate dehydrogenase. Reactions are conducted at 37°C. The reduction of NAD, which is directly proportional to ketolase activity, is followed by monitoring the increase at 340 nm. In addition, generation of acetyl-phosphate can be measured.

### Example C-7: Determining transketolase reaction with further substrates

Transketolase activity is measured by using a conventional enzyme-linked method under conditions in which coupling enzymes are not limiting. Reactions are initiated by the addition of transketolase protein to an otherwise complete reaction mix of 100 mmol/L Tris-Cl (pH 7.5), 5 mmol/L acetaldehyde, 5 mmol/L pyruvate, 1,2 mmol/L MgCl₂. The reaction leads to 3-hydroxybutanon (acetoin) and CO₂. Reactions are conducted at 37°C. Transketolase activity is measured by HPLC-chromatography.

Further substrates can be:
(a) Formaldehyde and pyruvate leading to hydroxyaceton and CO₂.
(b) Glycerinaldehyde and pyruvate leading to 1-desoxyxylulose and and CO₂.

### (G) Determining the in vivo TKTL1 reaction leading to lactate production

In vivo assays for identification of TKTL1 inhibitors based on lactate production

Cell lines, which can be tested are, e.g., glioblastoma cell line LN18, colon cancer cell line HT29, breast cancer cell line MCF7. Cell lines have to be grown in media containing 2 mg/ml glucose. Glucose consumption and lactate production has to be determined for 5 days. Every day the glucose and lactate content in the media is tested. As a control, glioblastoma cell line LN229 could be used, which does not show high glucose consumption and a high lactate production rate.

### Example 5: Diagnosis of neurodegenerative diseases

Fibroblast cell lines, foreskin fibroblasts or leukocytes from healthy subjects and patient with Alzheimer's disease, or other neurodegenerative diseases were analyzed for TKTL1 abnormalities by means of ELISA, electrofocusing gel analysis, 2D-gel electrophoresis and immunostaining. The ELISA experiments were performed by different ELISA approaches. One type of ELISA represents a typical ELISA, where catching or detecting antibody is directed against a certain protein. The other type of ELISA used consisted of an antibody directed against one protein and an antibody against another protein. An example for type 1 ELISA is the combination of TKTL1 antibody JFC12T10 and TKTL1 antibody JFC10T9. JFC12T10 detects an epitop of the TKTL1 protein, and does not cross react with TKT or TKTL2. JFC10T9 detects another epitop of TKTL1. Using the ELISA JFC12T10/JFC10T9 TKTL1 protein can be detected and measured. An example for Type 2 ELISA is antibody JFC12T10 directed against TKTL1 and antibody JFC11D8 directed against DNaseX. Using this ELISA the protein interaction of TKTL1 and DNaseX can be determined. Both types of ELISA reactions were performed with samples from healthy persons and patients. One type of sample consisted of bodyfluids like serum and was directly analysed for the presence of proteins and protein interactions. Another type of analysis was performed using an antibody (e.g. JFC12T10 or JFC11D8) coupled to carbo-link. Using affinity purification procedures we isolated multi-protein complexes of cells (derived from cell culture or native tissues). The multi-protein complexes were analysed by electrofocusing or 2D-gel electrophoresis followed by immunostaining or determination of enzymatic activity (e.g. transketolase two- or one-substrate reaction; DNase test, GAPDH activity). Using these assays, protein isoforms could be identified specifically present in patients with neurodegenerative diseases like AD. In patients with neurodegenerative disorders like AD patients, TKTL1 variants have been detected with high alkaline pI, lower two- or one-substrate reaction, and lower thiamin affinity. Additionally, using standard PAGE smaller protein isoforms and a higher amount of smaller protein in comparison to full length TKTL1 were detected in intact cells or cell extracts from those patients compared to healthy persons. Furthermore reduced two- or one-substrate reaction of TKTL1, or lower thiamin affinity of TKTL1 has been observed in healthy persons which later on (month and years later) showed neurodegenerative disease like AD. The observed TKTL1 variants lead to reduced sugar metabolism in cells. These reduced sugar metabolism lead to enhanced AGE formation and AGE formation lead to high molecular protein aggregates and cell death. This unwanted cell death of cells necessary for proper brain function, is an important cause for these neurodegenerative diseases. To identify individuals which do have TKTL1 variants with a reduced two- or one-substrate reaction or lower thiamin affinity, TKTL1 antibodies were established, which can be used to isolate TKTL1 proteins from samples to be tested (e.g. JFC12T10). Those samples can be body fluids (e.g. serum) or cell samples (e.g. proteins of fibroblasts or leukocytes). TKTL1 antibodies coupled to ELISA plates catch the TKTL1 proteins and after washing away TKT and TKTL2 proteins, the (trans-)ketolase two- or one substrate reaction can be enzymatically determined in a coupled enzymatic reaction by e.g. building the reduced NADH (the enzymatic assays are described above). Similarly the enzymatic reaction was performed at different concentrations of thiamin. By reducing the thiamine level in the assay TKTL1 variants were identified in patients with neurodegenerative diseases with a reduced affinity for thiamine. Using this approach of ELISA and enzymatic analysis, TKTL1 variants can be identified which predispose to neurodegenerative diseases at a timepoint before signs of neurodegenerative diseases are present. This can be exploited for a prevention of neurodegenerative diseases e.g. by appliccation of better soluble thiamine derivates like benfotiamine or a diet with reduced levels or certain types of sugars (e.g. glucose). In addition to the identification of TKTL1 variants with a reduced two- or one-substrate reaction or lower thiamin affinity, TKTL1 variants with a reduced solubility or TKTL1 variants present in high molecular weight complexes were identified in neurodegenerative disease patients like AD. The inventors establised TKTL1 specific antibodies specifically reacting with TKTL1 variants present in high molecular weight complexes in nuclei of patients with neurodegenerative disease patients like AD (JFC7T4). Using ELISA reaction or immunohistochemical stainings, those disease specific TKTL1 variants can be identified in body fluids (e.g. serum), or in tissue samples (e.g. leukocytes, fibroblasts, biopsies). Furthermore in combination with antibodies directed against other proteins present in those multi-protein complexes, ELISA can be performed detecting the presence of protein interactions. Such an type 2 ELISA consisting of TKTL1 antibody JFC8T7 and DNaseX antibody JFC7D4 identified a protein interaction of TKTL1 and DNaseX, specific for cells going into apoptosis. Another type 2 ELISA consisting of TKTL1 antibody JFC8T7 and GAPDH antibody JFC3G6 identified a protein interaction of TKTL1 and GAPDH, specific for cells going into apoptosis. The presence of these protein complexes can be expoited for the detection and therapy of neurodegenerative diseases. The identification of such protein interactions between TKTL1 and other proteins like GAPDH, DNaseX, and ph-Akt can be exploited for the isolation of antiapoptotic compounds. Those compounds can be used as pharmaceutical agents for the treatment of neurodegenerative diseases. Compounds specifically binding to TKTL1 can be identified by affinity labeling, and e.g. by means of BIAcore technology. Antiapoptotic effect can be detected using reduced programmed cell death (visualized by e.g. apoptotic ladder, caspase-3, annexin). TKTL1 and GAPDH are tightly bound to each other. The TKTL1 (trans-)ketolase reaction cleaving sugars like X5P leads to the production of GAP. As GAPDH is tightly bound to TKTL1, the produced GAP is directly used from GAPDH which lead to the production 1,3-phosphoglycerate concomittant with the reduction of NAD⁺ to NADH + H⁺. For the isolation of small compounds inhibiting TKTL1 and GAPDH interactions different NAD⁺ concentrations should be used since the binding of some compounds is depend on the concentration of NAD⁺. Another type of protein interaction was detected using antibody JFC12T10 as sole antibody. If the TKTL1 protein is present as a single protein, no ELISA reaction should work if just one antibody is used as catching and detecting antibody. In case of TKTL1 antibody JFC12T10 can be used as catching and detecting antibody. Therefore, using this antibody protein interactions of TKTL1 and another TKTL1 protein can be detected. As some of the TKTL1 protein isoforms miss N-terminal protein sequences dimers consisting of TKTL1 and TKTL1 can be discriminated into homo- and hetero TKTL1-dimers. Some of the dimers consists of full length TKTL1 protein bound to another full length TKTL1 protein (TKTL1 homodimer). Some of the dimers consists of a full length TKTL1 protein and a smaller TKTL1 isoform, where the N-terminus is missing. The discrimination can be performed using TKTL1 antibodies located at different sites within the TKTL1 protein. E.g. N-terminal located TKTL1 antibodies can be used with C-terminal located antibodies and the result of this ELSIA can be compared with an ELISA using only C-terminally located antibodies. The ratio between those two ELISA results can be used for determined in healthy and patients.

### Example 6: Screening methods for drug candidates

### (A) Assays for single compound testing

Cell lines (e.g., as described above) should be grown with and without the compound to be tested. As synthetic test compounds, for example, thiamin, oxythiamin, p-hydroxyphenylpyruvate, pyrithiamin, amprolium, 2-methylthiamin, benfooxytiamine, benfotiamine, 2-methoxy-*p-*benzochinon (2-MBQ) und 2,6- dimethoxy-p-benzochinon (2,6-DMBQ), genistein, and flavonols as e.g. quercetin, catechins, nitrilosides and anthocyanins or derivatives of them can be used.

Derivatives of the above listed compounds can be generated by substituting or adding one or more of the following groups:
linear and branched (C₁-C₁₂) aliphatic alkyl groups, substituted with at least one group chosen from OH, NH₂, SH, CN, CF₃, halogen, CONHR⁵, COOR⁵, OR⁵, SR⁵, SiOR⁵, NHR⁵, aliphatic (C₃-C₆) rings, and aromatic (C₃-C₆) rings, wherein R⁵ is chosen from linear and branched (C₁-C₄) alkyl groups,
aryl groups,
natural polymers, synthetic polymers, and copolymers, said polymers and copolymers carrying at least two groups chosen from: hydroxyl, carboxylate, primary amine, secondary amine, tertiary amine, thiol, and aldehyde;
a hydrogen atom, a halogen atom, CF₃, OH, OCF₃, COOH, R⁷, OR₇, and OCOR⁷, wherein R⁷ is chosen from linear and branched (C₁₋C₄) aliphatic alkyl groups;
a monohalogenated and polyhalogenated linear and branched (C₁-C₄) alkyl groups, and from aryl groups, wherein the aryl groups are optionally substituted with at least one group chosen from OH, NH₂, SH, CN, CF₃, halogen, COOH, CONHR⁸, COOR⁸, OR⁸, SR⁸, and NHR⁸, wherein R⁸ is chosen from linear and branched (C₁-C₁₂) alkyl radicals; and
from linear and branched (C₁-C₄) alkyl groups, and a CF₃ group.

Natural products, or extracts or fractions thereof can also be used to identify compounds for activating or inhibiting TKTL1 enzymatic activity, e.g., fermented wheat germ extract AVEMAR or apple extracts. Substrates or substrate-analogues specific for TKTL1 can be used to accelerate or inhibit TKTL1 enzymatic activity. Reactions specific for the TKTL1 protein isoforms can be exploited to inhibit or activate TKTL1 enzyme activities.

Compounds which inhibit the TKTL1 enzymatic activity can be used to prevent obesity.

Thus, compounds can be identified which will lead to a reduced glucose consumption or lactate production. Such compounds are, e.g., useful for reducing obesity, for reducing or inhibiting spermatogenesis, lactate production in sperms (leading to a matrix degradation in uterus) thus can be applied as contraceptives.

Moreover, compounds can be identified which will lead to an enhanced glucose consumption or lactate production. Such compounds can be used, e.g., for accelerating wound healing and bone repair, for reducing and normalizing blood glucose levels in diabetes mellitus patients, for preventing or reducing pathological alterations in small and large vessels in diabetes mellitus patients, for reducing retinopathies or neuropathies in diabetes mellitus patients and for inhibiting or preventing neurodegenerative diseases like Alzheimer disease, Wernicke-Korsakoff syndrome, Huntington disease, and Morbus Parkinson.

### (B) Assays for determining compounds influencing the protein-protein interactions of the mutated TKTL1 protein isoforms

Protein-protein interactions play a role both in regulating enzymatic activity and in signal transduction pathways that regulate cellular function. The number of small molecules protein-protein interaction inhibitors (SMPIIs) is growing rapidly. Living cells are continuously exposed to a variety of signals from their micro- and macro-environment. Many of these signals are detected by receptors present on the cell surface, and are then processed and transduced by intracellular signalling cascades. Because the ultimate site of action in a signalling cascade is often far from the cell surface, an inherent feature of intracellular signalling pathways is the requirement that proteins translocate from one position to another within the cell. These translocations, and thus cell signalling and response, depend critically on protein-protein interactions that mediate protein translocation through the intracellular space.

As an example of a typical signal transduction pathway involving protein translocation, the signalling and protein translocation steps involved in the cellular response of the phosphatidylinositol 3 kinase (PI3K) pathway to a growth factor such as insulin is depicted. This pathway influences and is influenced by TKTL1.
1. Insulin binds to and activates its receptor at the cell surface. Upon activation, the receptor recruits adaptor proteins and activates intracellular signalling molecules including PI3K.
2. Activated PI3K increases the plasma membrane concentration of the lipid phosphatidylinositol 3,4,5-triphosphate (PIP3).
3. PIP3 in the plasma membrane provides docking sites for protein kinases including Akt1/PKBa and PDK1; Akt is activated by PDK1 only when both are docked at the membrane. This translocation step is an absolute requirement for Akt activation.
4. Once activated by PDK1 at the plasma membrane, Akt is free to diffuse back into the cell interior, where it can phosphorylate substrate such as the transcription factor Forkhead (FKHR, FOXOA1).
5. Unphosphorylated FKHR normally resides in the nucleus, where it modulates genes involved in cell cycle arrest and apoptosis. However, once phosphorylated by Akt1, FKHR translocates to the cytoplasm, where it can no longer modulate target genes.

Protein-protein interactions and translocations are involved at each of these steps, notably for Akt1 and Forkhead. Thus, a signal initiated by the binding of insulin to a cell surface receptor modulates the transcription of genes involved in cellular growth and survival via a sequential cascade of protein translocation events. The therapeutic relevance of this becomes clear when one considers that altered signalling responses are often key distinguishing features between cells in normal and diseased tissues.

### (C) Assays for small-molecule protein-protein interaction inhibitors

Historically, large peptides and natural products have been considered the primary compound classes capable of modulating protein-protein interactions. However, there is growing evidence in the literature and from screening initiatives to suggest that small molecules can also modulate the interactions responsible for protein-protein complexes. These compounds may act either directly - via inhibition at the protein-protein interface - or indirectly - via binding to an allosteric site and induction of a conformational change of the target protein or an associated molecule.

Traditional small molecule drug discovery focuses primarily on the activity of compounds against purified targets, such as binding to cell-surface receptors or inhibition of the catalytic activity of enzymes. While these approaches have led to the development of a large number of useful drugs, they clearly have limitations. Because of the complex network environment in which intracellular signalling occurs, it is advantageous to screen compounds in living cells to reproduce the pathway and network context in which the drug will eventually have to act. When employed as part of a pathway screening strategy, cell-based translocation assays offer an opportunity to discover and progress entirely new classes of compounds that act primarily by modulating protein interactions.

Cell-based assays that monitor the intracellular behaviour of target molecules, rather than binding or catalytic activity of purified proteins, can now be used in high-throughput screens to discover and profile SMPPIIs.

Known TKT genes encode a single protein with enzymatic activity, whereas TKTL1 transcripts and proteins different in size have been detected. Furthermore, part of the TKTL1 protein(s) is present in the nucleus of cells. Therefore the one gene / one protein / one function relationship is wrong for the TKTL1 gene. Known transketolases are homodimers of two full length proteins harbouring all typical invariant transketolase amino acid residues. The transketolase-like gene encoded TKTL1 protein isoforms build TKTL1 homo/heterodimers and TKT/TKTL1 heterodimers. The expression of TKTL1 protein isoforms - even an enzymatically non-active - influences the enzymatic activity of a TKT protein as part of a TKT/TKTL1 heterodimer. A molecular switch and a proton wire synchronizes the active sites in TKT/TKTL1 heterodimers and TKTL1/TKTL1 homo- and heterodimers.

Another type of protein interaction is present as TKTL1 protein isoforms are part of a multi-protein complex. TKTL1 proteins are bound to transketolase unrelated proteins like GAPDH, DNaseX (DNA acc. No. X90392; protein acc. No. CAA62037), (phosphorylated-)Akt, histon, histon acetylase, actin binding protein, and amyloid precursor protein (APP).

The presence or binding of each member of the multi-protein complex changes. The changes are influenced by the translocation of cytoplasmic localized proteins to the nucleus. Once arrived in the nucleus, the former cytosolic proteins do exerts functions different to the function within the cytoplasm. We have detected a translocation of DNaseX from the cytoplasm to the nucleus in apoptotic and tumor cells (Fig. 13,14). We have also detected a translocation of ph-Akt from the cytoplasm to the nucleus in tumor cells (Fig. 6-8). A translocation of GAPDH has been detected in apoptotic neuronal cells. The release from cytoplasmic binding sites or the new synthesis of proteins, which are directly translocated into the nucleus, leads to multi-protein complexes which are inducing apotosis. This apoptosis is the basis for the death of cells, e.g. neurons in brains of patients with neurodegenerative diseases. In tumor cells the sucide molecul DNaseX is present in the nucleus, but exerts no DNase activity, which would lead to apoptosis and cell death of the tumor cells. Instead, binding to this multi-protein complex leads to inactivation of DNaseX in tumor cells. Therefore apoptosis is blocked. In neurodegenerative diseases DNaseX, GAPDH and TKTL1 lead to apoptosis of cells, cells which should not die. The unwanted apoptosis lead to the severe effects.

Bound proteins in the multi-protein complex were affinitiy-purified from cell lines using antibodies directed against TKTL1, DNaseX, ph-Akt, and GAPDH. Binding to certain proteins was assed by ELISA technique, using e.g. the combination of TKTL1 and GAPDH antibodies; the combination of TKTL1 and DNaseX antibodies; the combination of TKTL1 and ph-Akt antibodies; the combination of TKTL1 and TKT antibodies; the combination of TKTL1 and TKTL2 antibodies..

### (D) In vivo high-throughput screen to discover and profile SMPPIIs for influencing protein-protein interactions of TKTL1 protein isoforms

SMPPIIs can be identified which influence the generation of TKTL1 homo/heterodimers and TKT/TKTL1 heterodimers and the interaction to other proteins of the multi-protein complex. SMPPIIs can be identified which influence the generation of TKTL1 protein interactions with such other proteins e.g. DNaseX, GAPDH or amyloid beta peptide (A beta). SMPPIIs can be identified which influence the generation of TKTL1 protein aggregates.

SMPPIIs can be identified which influence the protein-protein interaction with other proteins and the following generation of protein aggregates e.g. GAPDH or amyloid beta peptide (A beta). SMPPIIs can be identified which influence the translocation of TKTL1 protein isoforms e.g. translocation from cytoplasm to nucleus.

The altered substrate specificity and reaction modus of the TKTL1 enzyme can be used for the destruction of cells or tissues with an enhanced TKTL1 enzyme activity. Application of a nontoxic substrate can be applied to patients with enhanced TKTL1 enzyme activity. Cells with an enhanced expression of TKTL1, harbor a gene product (TKTL1 enzyme) which targets the cells for selective killing. Those cells, which show an enhanced TKTL1 enzymatic activity, convert the nontoxic substrate into a toxic drug by rendering the cells sensitive to a nontoxic prodrug or a chemotherapeutic agent, thereby eliminating unwanted cells. This strategy of killing unwanted cells can be e.g. applied for epithelial cell (head and neck, oesophagus, gastric, colon and rectum and urothelial cells) by administering nontoxic prodrugs e.g. in food.

### (E) Mutations within the TKTL1 gene have been detected leading to TKTL1 protein isoforms with different isoelectric properties and reduced affinities for thiamine.

A test can be performed identifying mutations within the TKTL1 gene by DNA-based methods. A test can be performed by isolating TKTL1 protein isoforms using a monoclonal antibody specific for the TKTL1 protein(s). The antibody could be attached to microtiter plates. Serum or other samples could be analyzed and the TKTL1 protein isoform can be isolated form these specimens. A standardized enzymatic transketolase test could be performed allowing the determination of transketolase acitivity or Km-values for thiamine. Using this procedure, individuals with reduced TKTL1 activities could be identified prior to the beginning of the disease e.g. diabetes mellitus, Wernicke-Korsakoff syndrome, Huntington disease. Those patients should be treated with a TKTL1 activator compound.

An in vivo assay with cells can be performed for screening small compound inhibiting the translocation to the nucleus or the aggregation of TKTL1 protein within the nucleus, as monitored e.g. by (in vivo) immunohistochemical methods. Cells can be analysed for the presence of high molecular weight complexes harbouring TKTL1 or the presence of protein complexes with reduced solubility. The above mentioned in vivo assays can also be performed using a TKTL1-GFP fusion protein.

## Claims

1. A method for the diagnosis of a disease associated with a dysfunction of a TKTL1 protein in a biological sample obtained from a patient, wherein said method comprises determining the activity or level of the TKTL1 protein, the cellular localization of the TKTL1 protein, its aggregation status and/or its dimerization status, wherein an enhanced or decreased level of activity, an abnormal cellular localization of the mutated TKTL1 protein, aggregation status and/or dimerization status compared to a control is indicative of such disease, **characterized in that** the disease is a neurodegenerative disease or diabetes.

2. The method of claim 1, wherein said TKTL1 protein is **characterized by** a modified substrate specificity and/or modified reaction modus.

3. The method according to claim 1 or 2, wherein the biological sample is a body fluid, a secretion, a smear, a biopsy, a liquid containing cells, lysed cells, cell debris, peptides or nucleic acids.

4. The method according to claim 3, wherein the sample is serum, urine, semen, stool, bile, a biopsy or a cell- or tissue-sample.

5. The method according to any one of the claims 1-4, wherein determination is carried out on a polypeptide level.

6. The method according to any one of the claims 1-5, wherein determination is carried out on a nucleic acid level.

7. The method according to claim 6, wherein determination on the polypeptide level is carried out using a binding agent directed against TKTL1 polypeptides.

8. The method of claim 7, wherein the binding agent is an antibody, a fragment of an antibody, a peptidomimetic comprising an antigen binding epitope or a mini-antibody.

9. The method according to any one of the claims 5, 7 or 8, wherein determination is an immuno-cytochemical detection procedure.

10. The method according to claim 6, wherein at least one nucleic acid probe, hybridising to a TKTL1 nucleic acid is used for determination.

11. The method according to claim 10, wherein the probe is detectably labelled.

12. The method according to claim 11, wherein the label is a radioisotope, a bioluminescent compound, a chemiluminescent compound, a fluorescent compound, a metal chelate, or an enzyme.

13. The method according to any one of the claims 6 or 10-12, wherein the determination reaction comprises a nucleic acid amplification reaction.

14. The method according to claim 13, wherein the amplification reaction is PCR, LCR, SDA or NASBA.

15. The method according to any one of the claims 6 or 10-12, which is used for in-situ hybridisation.

16. The method according to any one of claims 1 to 15 which is used in the course of an in vivo or in vitro molecular imaging method.

17. The method according to any one of claims 1 to 16, wherein said disease is diabetes mellitus, retinopathy, neuropathy, endothelial cell damage, atherosclerosis, a neurodegenerative disorder, low or high body weight, or a condition associated with low or high level of blood sugar.

18. Use of a TKTL1 polypeptide, a TKTL1 encoding nucleic acid or an activator (agonist) of TKTL1 polypeptide or of expression of the TKTL1 encoding nucleic acid for the preparation of a pharmaceutical composition for the prevention or treatment of a disease which is associated with a decreased level of activity, an abnormal cellular localization of the mutated TKTL1 protein, aggregation status and/or dimerization status compared to a control.

19. Use according to claim 18, wherein said disease is diabetes mellitus, retinopathy, neuropathy, endothelial cell dysfuntion, atherosclerosis, a neurodegenerative disorder, obesity, or low or high body weight.

20. Use according to claim 19, wherein the neurodegenerative disorder is Alzheimer's disease, Parkinson's disease or a disease associated with an impaired function of central or peripheral nerve cells.

21. Use according to any one of claims 18 to 20, wherein said activator (agonist) of TKTL1 polypeptide is thiamine, benfotiamine or a derivative thereof.

22. Use according to any one of claims 18 to 20, wherein said TKTL1 encoding nucleic acid is a chimeric nucleic acid comprising a TKTL1 encoding nucleic acid or fragments thereof or said TKTL1 polypeptide is a fusion protein comprising a TKTL1 polypeptide or a fragment thereof.

23. Use of an inhibitor (antagonist) of TKTL1 polypeptide or an inhibitor of the expression of a TKTL1 encoding nucleic acid for the preparation of a pharmaceutical composition for the treatment of a disease associated with an enhanced level or activity, an abnormal cellular localization of the mutated TKTL1 protein, aggregation status and/or dimerization status compared to a control.

24. Use according to claim 23, wherein said disease is high body weight or conditions associated with low level of blood sugar.

25. Use according to claim 23, wherein high body weight is adiposity.

26. Use according to claim 23 for contraception.

27. Use according to any one of claims 23 to 26, wherein said inhibitor of TKTL1 polypeptide is oxythiamine, benfooxytiamine, hydroxypyruvate, pyruvate, p-hydroxyphenylpyruvate, pyrithiamin, amprolium, 2-methylthiamin, benfotiamine, benfooxytiamine, 2-methoxy-*p-*benzochinon (2-MBQ) und 2,6-dimethoxy-*p*-benzochinon (2,6-DMBQ), genistein, and flavonols as e.g. quercetin, catechins, nitrilosides, anthocyanins; or derivatives thereof.

28. Use according to any one of claims 18 to 27, wherein prevention or treatment is vaccination therapy or immunotherapy.

29. Use of compound for the treatment of a disease associated with an enhanced or decreased level or activity, an abnormal cellular localization of the mutated TKTL1 protein, aggregation status and/or dimerization status compared to a control, wherein said compound is selected from the group consisting of oxythiamine, benfooxytiamine, hydroxypyruvate, pyruvate, p-hydroxyphenylpyruvate, pyrithiamin, amprolium, 2-methylthiamin, benfotiamine, benfooxytiamine, 2-methoxy-*p-*benzochinon (2-MBQ) und 2,6-dimethoxy-p-benzochinon (2,6-DMBQ), genistein, and flavonols as e.g. quercetin, catechins, nitrilosides, anthocyanins, and derivatives thereof; and said compound is identified by a method comprising the following steps:
(a) contacting a TKTL1 polypeptide as defined in the preceding claims or a cell expressing said polypeptide in the presence of components capable of providing a detectable signal in response to a biological activity; and
(b) detecting presence or absence of a signal or increase of the signal generated from said biological activity, wherein the absence, decrease or increase of the signal is indicative for a putative drug.

30. The use according to claim 29, wherein said biological activity is transketolase or ketolase activity, including the forward as well as the backward reaction.


SEQUENCE LISTING

<110>  Coy, Johannes

<120>  Therapeutic and diagnostic uses of TKTL1 and inhibitors and
       activators thereof

<130>  C 1031EP

<140>  EP 05004930
<141>  2005-03-07

<160>  1

<170>  PatentIn version 3.2

<210>  1
<211>  23
<212>  DNA
<213>  artificial

<220>
<223>  probe

<400>  1
tctcatcaca agcagcacag gac                                              23